# EUROPEAN PATENT APPLICATION

(11) **EP 2 570 433 A1**
(43) Date of publication of application: **20.03.2013**
(21) Application number: 12175602.7
(22) Date of filing: 14.11.2003
(51) Int. Cl.: C07K 16/12, A61K 39/40, G01N 33/577, G01N 33/68

(54) **Neutralizing human antibodies to anthrax toxin generated by recall technology**

(62) Divisional of application: 03816309.3
(71) Applicant: Emergent Product Development Gaithersburg Inc., Gaithersburg, MD 20879 (US)
(72) Inventor: Kang, Angary S, Encinitas, CA 92024 (US); Wang, Fei, San Diego, CA 92129 (US); Jiang, Ivy, San Diego, CA 9213 (US); Sawada-Hirai, Ritsuko, San Diego, CA 92130 (US); Scholz, Wolfgang, San Diego, CA 92129 (US); Morrow, Phillip R, (deceased) (US)
(74) Representative: Jappy, John William Graham

(57) **Abstract**

A highly efficient method for generating human antibodies using recall technology is provided. In one aspect, human antibodies which are specific to the anthrax toxin are provided. In one aspect, human peripheral blood cells that have pre-exposed to anthrax toxin are used in the SCID mouse model. This method results in high human antibody titers which are primarily of the IgG isotype and which contain antibodies of high specificity and affinity to desired antigens. The antibodies generated by this method can be used therapeutically and prophylactically for preventing or treating mammals exposed to anthrax. Methods for diagnosis and methods to determine anthrax contamination are also described.

## Description

### Background of the Invention

### Field of the Invention

The present invention relates to the field of fully human monoclonal antibodies, method of making same, and their use in preventive and therapeutic applications in anthrax. More particularly, antibodies have binding specificity for anthrax protective antigen (PA) toxin are provided.

### Description of the Related Art

Anthrax is a zoonotic soil organism endemic to many parts of the world. The *B*. anthracis organism was one of the first biological warfare agents to be developed and continues to be a major threat in this regard. Although vaccine strains have been developed, currently there are concerns regarding their efficacy and availability. A passive immunization strategy may be useful in conferring medium-term protection, and can also have benefits for non-immunized patients who seek treatment after the point at which antibiotic therapy alone is effective. Casadevall, A., Emerging Infectious Diseases, 8:8 (2002); Maynard, J. A et al., Nature Biotechnology, 20:597 (2002). After inhalation by mammals, *B*. anthracis spores germinate in the alveolar macrophages, then migrate to lymph nodes where they multiply and enter the bloodstream. The vegetative bacteria excrete the tripartite exotoxin that is responsible for the etiology of the disease. In addition to capsule, virulent strains of *Bacillus anthracis* secrete a set of three distinct antigenic protein components: protective antigen (PA), edema factor (EF), and lethal factor (LF). PA can bind either LF or EF, forming lethal toxin (LeTx) or edema toxin (EdTx). Collectively these two toxins are seen as a complex exotoxin called anthrax toxin. Each component of the toxin is a thermolabile protein with a molecular weight exceeding 80kDa. Edema factor (EF) is an adenylate cyclase that is responsible for the edema seen in anthrax infections. Lethal factor (LF) is a zinc-metalloprotease that is essential for the lethal effect of the anthrax toxin on macrophages. Protective antigen (PA) contains the binding domain of anthrax toxin, which binds to a recently identified receptor on the cell surface and allows translocation of LF or EF into the cell by endocytosis.

Evidence that the hu-PBL-SCID system can be used to obtain recall antibody responses dates from the original publication of the method by Mosier and co-workers. Mosier et al., Nature 335:256 1988. In this report, tetanus toxoid was administered to human PBL engrafted mice, and human antibodies to tetanus were found in the serum post-immunization. Since this original report, many investigators in various labs worldwide have used the hu-PBL-SCID system to examine aspects of the human recall antibody response to multiple antigens. Nonoyama, S. et al., J. Immunol., 151:3894 (1993); Walker, W. et al., Eur. J Immunol., 25:1425 (1995); Else, K. J., and Betts. C. J., Parasite Immunology 19:485 (1997). However, reports describing the generation of useful monoclonal antibodies from such engrafted mice have been sporadic. Satoh, N. et al., Immunology Letters 47:113 (1995); Duchosal, M. A. et al., Letters To Nature:258 (1991); Smithson, S. L. et al., Molecular Immunology 36:113 (1999); Coccia, M. A., and P. Brams, Amer. Assoc. Immunologists:5772 (1998); Nguyen, H. et al., Microbiol. Immunol. 41:901 (1997); and Uchibayashi, N. et al., Hybridoma 14:313 (1995).

Accordingly, there still remains a need for an effective method to produce human monoclonal antibodies that are specific to a particular antigen. Moreover, a need for a human monoclonal antibody specific to the anthrax toxin still remains.

### Summary of the Invention

It is an object of several embodiments of the present invention to provide antibodies that bind to the PA component of the tripartite anthrax exotoxin. These antibodies will provide protection either as single agents or combined in a cocktail. It is another object to provide a method to generate a series of fully human anti-anthrax PA toxin antibodies.

In one embodiment, a fully human monoclonal antibody or fragment thereof is disclosed, which specifically recognizes at least a portion of an anthrax exotoxin. In one variation, the portion of an anthrax exotoxin is selected from the group consisting of protective antigen (PA), lethal factor (LF) and edema factor (EF).

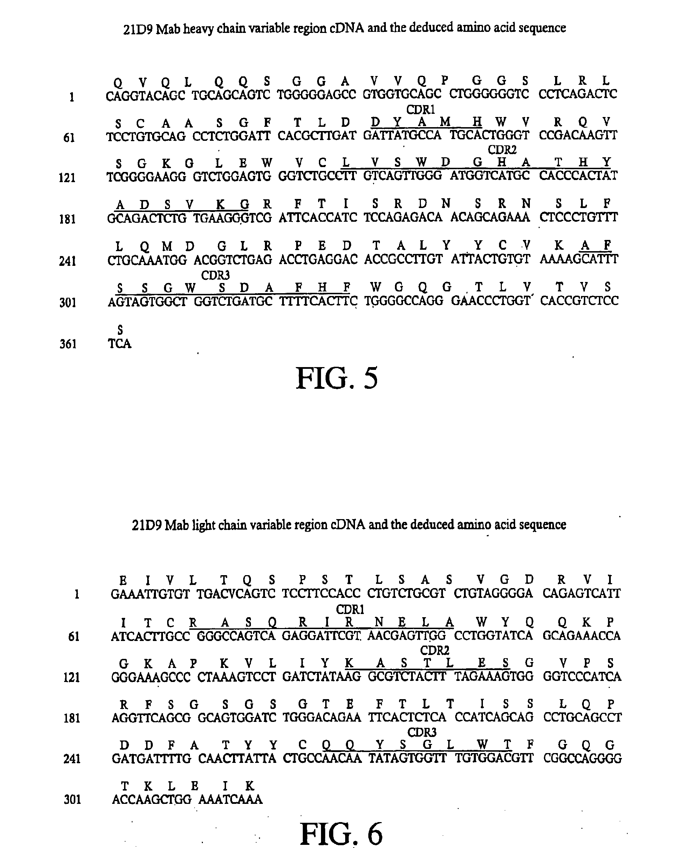

In a preferred embodiment, a fully human immunoglobulin or fragment thereof, that recognizes at least a portion of an anthrax exotoxin is disclosed, wherein the immunoglobulin or fragment thereof comprises an immunoglobulin heavy chain comprising the amino acid sequence shown in **FIG. 5****.** In a variation, the immunoglobulin or fragment thereof is encoded by the nucleotide sequence shown in **FIG. 5****.**

In another embodiment, the immunoglobulin or fragment thereof comprises an immunoglobulin heavy chain comprising CDR1, CDR2, and CDR3; wherein the CDR1 is comprised of the amino acid sequence, as shown in **FIG. 5****;** the CDR2 is comprised of the amino acid sequence, as shown in **FIG. 5****;** and the CDR3 is comprised of the amino acid sequence, as shown in **FIG. 5****.** In a variation, the CDR1, CDR2, and CDR3 regions of the immunoglobulin are encoded by the nucleotide sequences, as shown respectively in **FIG. 5****.**

In another preferred embodiment, a fully human immunoglobulin or fragment thereof is disclosed, that recognizes at least a portion of an anthrax exotoxin, wherein the immunoglobulin or fragment thereof comprises an immunoglobulin light chain comprising the amino acid sequence shown in **FIG. 6****.** In a variation, the immunoglobulin or fragment thereof is encoded by the nucleotide sequence shown in **FIG. 6****.**

In another embodiment, the immunoglobulin or fragment thereof comprises an immunoglobulin light chain comprising CDR1, CDR2, and CDR3; wherein the CDR1 is comprised of the amino acid sequence, as shown in **FIG. 6****;** the CDR2 is comprised of the amino acid sequence, as shown in **FIG. 6****;** and the CDR3 is comprised of the amino acid sequence, as shown in **FIG. 6****.** In a variation, the CDR1, CDR2, and CDR3 regions of the immunoglobulin are encoded by the nucleotide sequences, as shown respectively in **FIG. 6****.**

In one preferred embodiment of the present invention, the fully human immunoglobulin or fragment thereof is a single chain that recognizes at least a portion of an anthrax exotoxin.

In one preferred embodiment, a fully human immunoglobulin or fragment thereof is disclosed, that recognizes at least a portion of an anthrax exotoxin, wherein the immunoglobulin or fragment thereof comprises an immunoglobulin heavy chain comprising at least one complementary determining region selected from the group consisting of CDR1, CDR2 and CDR3; wherein the CDR1 is comprised of the amino acid sequence, as shown in **FIG. 5****;** the CDR2 is comprised of the amino acid sequence, as shown in **FIG. 5****;** and the CDR3 is comprised of the amino acid sequence, as shown in **FIG. 5****.**

In one preferred embodiment, a fully human immunoglobulin or fragment thereof is disclosed, that recognizes at least a portion of an anthrax exotoxin, wherein the immunoglobulin or fragment thereof comprises an immunoglobulin light chain comprising at least one complementary determining region selected from the group consisting of CDR1, CDR2 and CDR3; wherein the CDR1 is comprised of the amino acid sequence, as shown in **FIG. 6****;** the CDR2 is comprised of the amino acid sequence, as shown in **FIG. 6****;** and the CDR3 is comprised of the amino acid sequence, as shown in **FIG. 6****.**

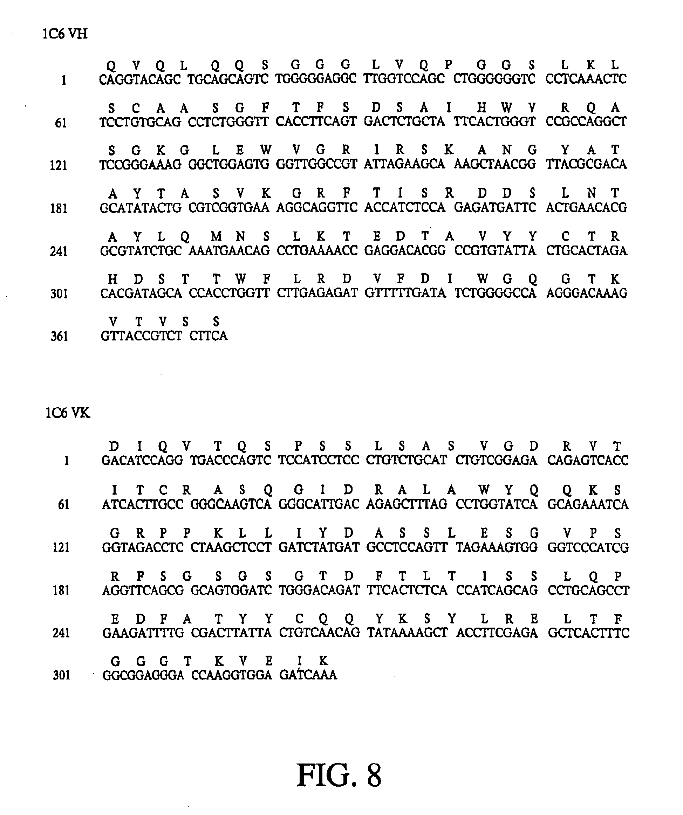

In another preferred embodiment, a fully human immunoglobulin or fragment thereof is disclosed, that recognizes at least a portion of an anthrax exotoxin, wherein the immunoglobulin or fragment thereof comprises an immunoglobulin heavy chain or light chain comprising the amino acid sequence shown in **FIG. 8****.**

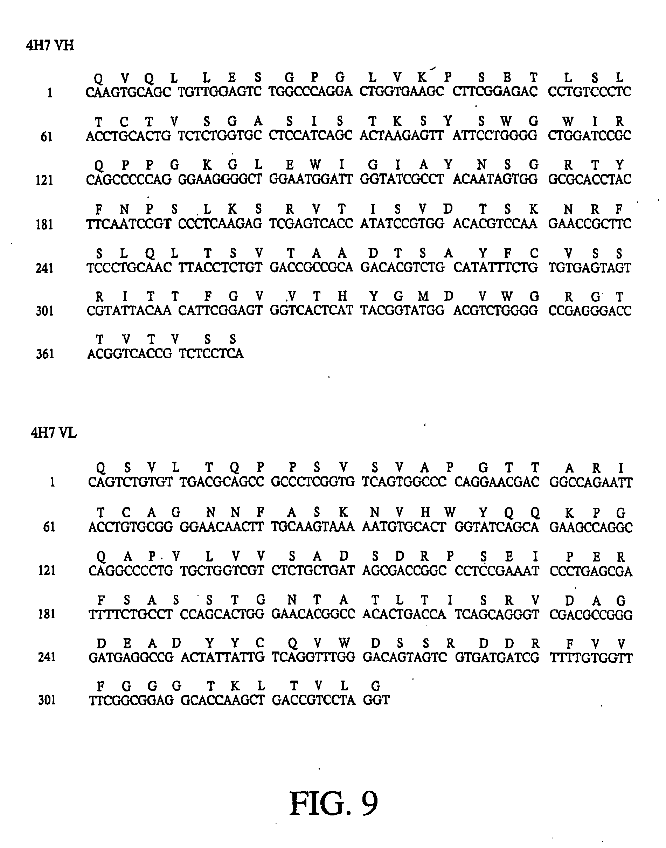

In another preferred embodiment, a fully human immunoglobulin or fragment thereof is disclosed, that recognizes at least a portion of an anthrax exotoxin, wherein said immunoglobulin or fragment thereof comprises an immunoglobulin heavy chain or light chain comprising the amino acid sequence shown in **FIG. 9****.**

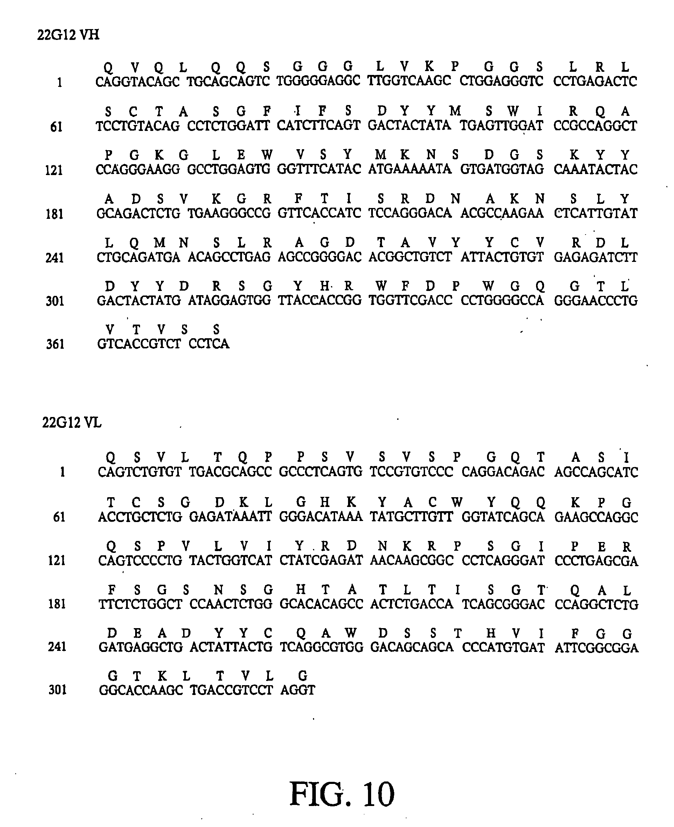

In another preferred embodiment, a fully human immunoglobulin or fragment thereof is disclosed, that recognizes at least a portion of an anthrax exotoxin, wherein said immunoglobulin or fragment thereof comprises an immunoglobulin heavy chain or light chain comprising the amino acid sequence shown in **FIG. 10****.**

In accordance with other embodiments of the invention the nucleotide sequences shown respectively in **FIG. 5** and **FIG. 6** are disclosed. These nucleotide sequences encode a heavy chain variable region and a light chain variable region, respectively, of a fully human immunoglobulin or fragment thereof, that recognizes at least a portion of an anthrax exotoxin.

In accordance with other embodiments of the invention the nucleotide sequences shown in **FIG. 8** are disclosed. These nucleotide sequences encode a heavy chain variable region and a light chain variable region, respectively, of a fully human immunoglobulin or fragment thereof, that recognizes at least a portion of an anthrax exotoxin.

In accordance with other embodiments of the invention the nucleotide sequences shown in **FIG. 9** are disclosed. These nucleotide sequences encode a heavy chain variable region and a light chain variable region, respectively, of a fully human immunoglobulin or fragment thereof, that recognizes at least a portion of an anthrax exotoxin.

In accordance with other embodiments of the invention the nucleotide sequences shown in **FIG. 10** are disclosed. These nucleotide sequences encode a heavy chain variable region and a light chain variable region, respectively, of a fully human immunoglobulin or fragment thereof, that recognizes at least a portion of an anthrax exotoxin.

In another preferred embodiment of the present invention, a method is disclosed for generating a fully human monoclonal antibody which specifically recognizes at least a portion of an anthrax exotoxin. The method comprises administering peripheral blood mononuclear cells from one or more human donors exposed to anthrax to an immuno-compromised animal; isolating at least one lymphocytic cell from the animal; and fusing the at least one lymphocytic cell with a hybridoma fusion partner, thereby generating a fully human monoclonal antibody.

In variations to the above method, the method may further comprise screening the generated antibodies; transforming at least a portion of said lymphocytic cells with EBV; characterizing the animal's immune response using a test bleed; administering one or more booster injections of anthrax antigen to the animal; administering one or more injections of anti-CD8 to the animal; and using a double selection method to select against undesirable cells, wherein the double selection method comprises using HAT selection or using ouabain.

In further variations to the method, the human donor is an anthrax-vaccinated donor and/or the human donor has been inadvertently exposed to anthrax.

In preferred embodiments, the animal is a SCID mouse.

Preferably, the hybridoma fusion partner is derived from a mouse myeloma MOPC21. In one embodiment, the hybridoma fusion partner is a myeloma. In another embodiment, the hybridoma fusion partner is P3x63Ag8.653.

In one preferred embodiment, the portion of an anthrax exotoxin is selected from the group consisting of PA, LF and EF.

In accordance with another preferred embodiment of the invention, a method is disclosed for inhibiting the assembly of the protective antigen (PA) of an anthrax exotoxin on receptors in a human. Preferably, the method comprises administering to such human the antibody of any of the immunoglobulins or fragments thereof described above, including those described in **FIGS. 5, 6****,** **8****,** **9****,** and **10****.**

A pharmaceutical composition for vaccinating a mammal against anthrax is disclosed in accordance with another embodiment of the invention. The pharmaceutical composition comprises the fully human monoclonal antibody of any of the immunoglobulins or fragments thereof described above, including those described in **FIGS. 5, 6****,** **8****,** **9****,** and **10****.**

A pharmaceutical composition for treating a mammal exposed to an anthrax exotoxin is disclosed in accordance with another embodiment of the invention. The pharmaceutical composition comprises the fully human monoclonal antibody of any of the immunoglobulins or fragments thereof described above, including those described in **FIGS. 5, 6****,** **8****,** **9****,** and **10****.**

In another embodiment, a method of vaccinating a mammal against anthrax is disclosed. The method comprises administering to the mammal an immunizing dose of the fully human monoclonal antibody of any of the immunoglobulins or fragments thereof described above, including those described in **FIGS. 5, 6****,** **8****,** **9****,** and **10****.**

A method of treating a mammal exposed to anthrax is also disclosed. The method comprises administering to the mammal a therapeutic dose of the fully human monoclonal antibody of any of the immunoglobulins or fragments thereof described above, including those described in **FIGS. 5, 6****,** **8****,** **9****,** and **10****.**

A method of identifying the presence of anthrax exotoxin in a sample is disclosed in accordance with another embodiment of the present invention. The method comprises contacting at least a portion of the sample with the fully human monoclonal antibody of any of the immunoglobulins or fragments thereof described above, including those described in **FIGS. 5, 6****,** **8****,** **9****,** and **10****;** and determining binding of anthrax exotoxin with the antibody, wherein the binding is an indicator of the presence anthrax in the sample.

A kit to identify the presence of anthrax exotoxin in a sample is also disclosed. The kit comprises the fully human monoclonal antibody of any of the immunoglobulins or fragments thereof described above, including those described in **FIGS. 5, 6****,** **8****,** **9****,** and **10****;** and an assay system to determine the binding of anthrax exotoxin with the antibody, wherein the binding is an indicator of the presence anthrax in the sample.

In another preferred embodiment of any of the immunoglobulins or fragments thereof described above, including those described in **FIGS. 5, 6****,** **8****,** **9****,** and **10****,** the anthrax exotoxin is tripartite. In a variation, the exotoxin is naturally-occurring or synthetic.

### Brief Description of the Drawings

Figure 1 shows a timeline of the engraftment of SCID mice with human PBMC from anthrax-vaccinated donors.
Figures 2A-H show anti-anthrax toxin levels in donor plasma compared to the engrafted mice.
Figure 3 shows testing of the presence of neutralizing PA bioactivity in donor and HuPBL-SCID engrafted mice sera.
Figure 4 shows a dose-response curve of the inhibition of anthrax PA toxin bioactivity with 21 D9 MAb.
Figure 5 shows the full nucleotide sequence and amino acid sequence of the 21 D9 MAb heavy chain variable region.
Figure 6 shows the full nucleotide sequence and amino acid sequence of the 21 D9 MAb light chain variable region.

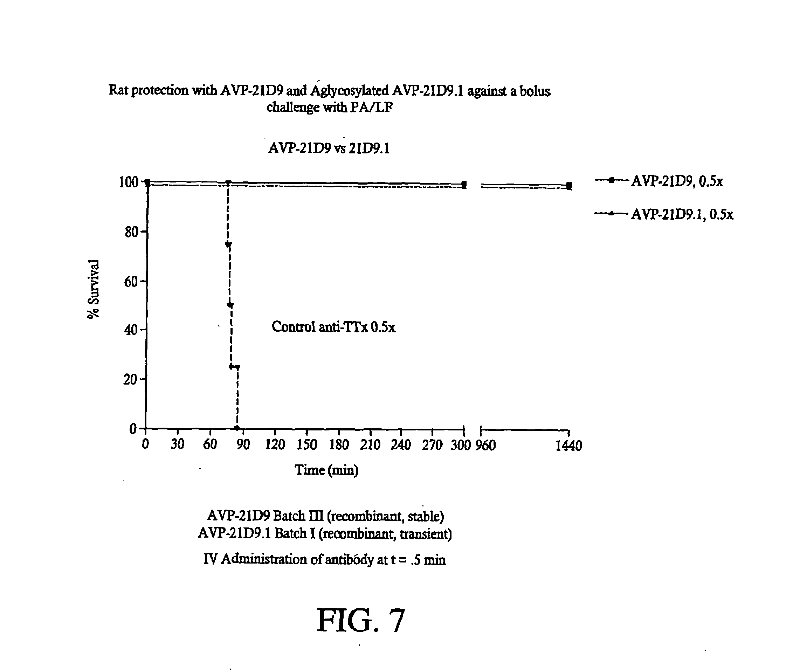

Figure 7 shows survival data in a rat protection model.
Figure 8 shows the full nucleotide sequence and amino acid sequence of the 1C6 Mab VH and VK chain variable regions.
Figure 9 shows the full nucleotide sequence and amino acid sequence of the 4H7 Mab VH and VL chain variable regions.
Figure 10 shows the full nucleotide sequence and amino acid sequence of the 22G12 Mab VH and VL chain variable regions.

### Detailed Description of the Preferred Embodiment

Antibodies which bind to one or more components of the tripartite anthrax exotoxin, the methods of making said antibodies, and the methods of using said antibodies are provided. In several embodiments, the antibodies provide protection either as single agents or combined in a cocktail. Anthrax, as defined herein, shall be given its ordinary meaning and shall also include the tripartite anthrax toxin, synthetic or naturally-occurring, and shall also be defined broadly to include one or more of the following components, synthetic or naturally-occurring: protective antigen (PA), lethal factor (LF) and edema factor (EF). Thus, antibodies to "anthrax" shall include antibodies to any portion of one or more components of the anthrax toxin. Moreover, as used herein, the singular forms "a", "an", and "the" include plural reference, unless the context clearly dictates otherwise. Thus, for example, a reference to "a host cell" includes a plurality of such host cells, and a reference to "an antibody" is a reference to one or more antibodies and equivalents thereof known to those skilled in the art.

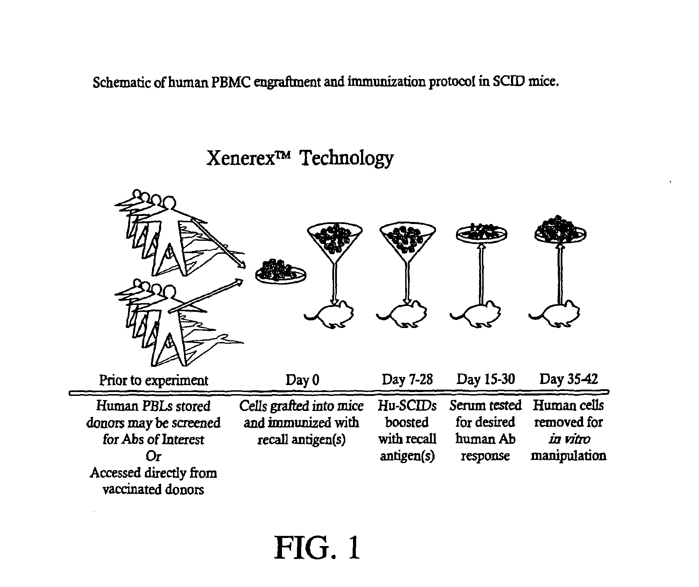

As shown generally in **FIG. 1****,** in one embodiment, a method of preparing a fully human monoclonal antibody which specifically recognizes at least a portion of the protective antigen (PA) of an anthrax exotoxin is provided. In one embodiment, this method includes obtaining peripheral blood mononuclear cells from human donors. After obtaining the peripheral blood mononuclear cells from donors, the blood cells are administered to an immuno-compromised animal. The lymphocytic cells are isolated and fused with a hybridoma fusion partner.

In a preferred embodiment, blood cells from donors who have been exposed to anthrax are obtained. Such exposure may have occurred naturally through exposure, or may have occurred by vaccination. Moreover, in one embodiment, exposure may have occurred decades, years or days prior to obtaining the donor's blood cells. In one embodiment, the "memory" of said exposure is captured or recalled and is selectably expanded by immunizing the engrafted SCID mice. Thus, in a preferred embodiment, said recall technology is used to generate human monoclonal antibodies. In one embodiment, the human donor has been vaccinated against anthrax. The use of human blood cells that have been "pre-exposed" to anthrax, or another target antigen, yields surprising and unexpected advantages. These advantages include the generation of antibodies with higher affinity, higher specificity, and more potent neutralization capabilities.

In another embodiment, unexposed or naïve blood cells are used. In one embodiment, the unexposed blood cells are exposed to anthrax ex vivo or in vitro, prior to engraftment in the immuno-deficient mouse. Thus, said initially unexposed cells are transformed into exposed cells and can be used in accordance with the recall technology described above.

In a preferred embodiment, peripheral blood mononuclear cells are obtained from a donor. In another embodiment, other cell types are obtained, including but not limited to lymphocytes, splenocytes, bone marrow, lymph node cells, and immune cells.

In one embodiment, the blood cells are administered to an immuno-compromised or immuno-deficient animal. In one embodiment, the animal is a SCID mouse.

In one aspect of the invention, the animal's immune response is characterized using a test bleed. In another embodiment, the generated antibodies are screened and isolated. In yet another embodiment, the lymphocytic cells are transformed with EBV. In one embodiment, one or more booster injections of anthrax antigen are administered to the immuno-compromised animal. In another embodiment, one or more injections of anti-human CD8 is administered to the animal. In yet another aspect, a double selection method to select against undesirable cells is used, including, but not limited to using HAT and ouabain. In one embodiment of the present invention, the hybridoma fusion partner is the mouse myeloma P3x63Ag8.653. In another embodiment of the present invention, the hybridoma fusion partner is derived from the mouse myeloma P3x63Ag8.653.

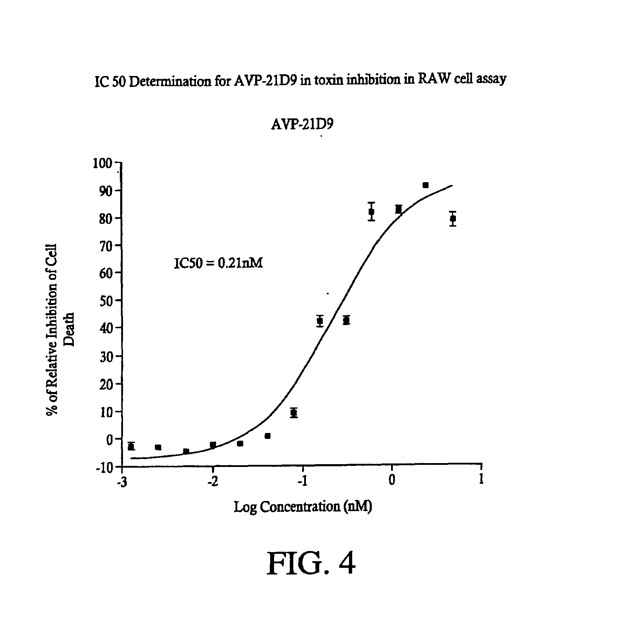

In one embodiment of the present invention, a series of human anti-anthrax PA toxin antibodies is provided. In one embodiment, a monoclonal antibody (IJ8:21D9, or "21D9") is provided. As illustrated in **FIG. 4****,** antibody 21D9 was effective in RAW cell assays in toxic inhibition. Antibody 21D9 was also shown to protect *in vitro* a mouse macrophage cell line from toxin challenge. The IC₅₀ of 21D9 was found to be in the picomolar range and in approximately equimolar stoichiometry with the input PA toxin. The equilibrium dissociation constant (K_{d}) as determined - by BiaCore analysis revealed this embodiment to bind antigen with high affinity in the picomolar range. Deduced amino acid sequence from the 21D9 hybridoma heavy and light chain cDNA allowed assignment to known VH and VL gene families, although significant mutation away from these germline sequences was also observed thereby indicating the occurrence of somatic hypermutation. In one embodiment, the mechanism by which 21 D9 provides protection is also provided. Antibody 21D9, and other antibodies described herein, can be used for human use in vivo for prophylaxis and treatment of Anthrax Class A biowarfare toxins. Thus, in several embodiments, a method for preventing anthrax infection is provided. In one embodiment, a method for vaccinating mammals to prevent anthrax infection is provided. In a further embodiment, a method to treat mammals who have been exposed to anthrax is provided.

In one embodiment, antibody 22G12, and methods of making and using same, are provided. In another embodiment, antibody 1 C6, and methods of making and using same, are provided In a further embodiment, antibody 4H7, and methods of making and using same, are provided.

Preferred embodiments provide a fully human monoclonal antibody that specifically binds to a component of an anthrax exotoxin or combinations of components thereof. The anthrax exotoxin can be in tripartite form; and the anthrax toxin can be naturally-occurring or synthetic. In a tripartite form, the anthrax exotoxin comprises protective antigen (PA), edema factor (EF), and lethal factor (LF).

In preferred embodiments, a monoclonal antibody is produced by rescuing the genes encoding antibody variable region from the antibody-producing cells and establishing stable recombinant cell lines producing whole IgG/kappa or IgG/lambda. In one embodiment, antibody-producing cells recovered from the immunized animal are subjected to cell fusion with an appropriate fusion partner. The resulting hybridomas are then screened in terms of the activity of the produced antibodies. The hybridomas subjected to selection are screened first in terms of the binding activity to a component of the tripartite anthrax exotoxin. In one embodiment, the hybridomas are selected based on ability to bind to PA, LF and/or EF proteins in an immunoassay, such as an ELISA test and also a bioassay. In one embodiment, the hybridoma shows protection in the bioassay. The cells from positive wells are used to isolate mRNA. From the mRNA, cDNA is reverse transcribed. Using either primers for the CH1 domain or the frame work 4 of the light chains and reverse primers the variable domains are PCR amplified.

In one embodiment, the amino acid sequences constituting the variable regions of the antibodies having a desired binding activity to PA, LF and/or EF and the nucleotide sequences encoding the same is provided. Several embodiments provide immunoglobulin variable regions containing the amino acid and nucleotide sequences shown in FIGS. 5, 6, 8, 9, and 10. FIG. 5 shows the nucleotide sequence and amino acid sequence of the 21D9 MAb heavy chain variable region. **FIG. 6** shows the 1 nucleotide sequence and amino acid sequence of the 21D9 MAb light chain variable region. In a further embodiment, cDNA encoding the immunoglobulin variable regions containing the nucleotide sequences shown in **FIG. 5** and **FIG. 6** is provided. In one embodiment, these amino acid sequences or cDNA nucleotide sequences are not necessarily identical but may vary so long as the specific binding activity to PA, LF and/or EF is maintained. In another embodiment, variation in nucleotide sequence is accommodated. As will be later described, in several embodiments, the site corresponding to CDR is highly variable. In the CDR region, even entire amino acids may vary on some occasions.

In one embodiment, each immunoglobulin molecule consists of heavy chains having a larger molecular weight and light chains having a smaller molecular weight. The heavy and light chains each carries a region called "a variable region" in about 110 amino acid residues at the N-terminus, which are different between the molecules. Variable regions of a heavy chain and a light chain are designated VH and VL, respectively. The antigen-binding site is formed by forming a dimer through electrostatic interaction between the heavy chain variable region VH and the light chain variable region VL. The variable region consists of three complementarity determining regions (CDRs) and four frameworks. The CDR forms a complementary steric structure with the antigen molecule and determines the specificity of the antibody. The three CDRs inserted between the four framework regions (FRs) are present like a mosaic in the variable region (E. A. Kabat et al., Sequences of proteins of immunological interest, vol. I, 5th edition, NIH Publication, 1991). The amino acid sequences of FRs are well conserved, but those of CDR are highly variable and may thus be called hypervariable regions. Among the amino acid sequences of the antibody specifically recognizing PA, LF and/or EF, a CDR that determines the binding activity to antigens is provided in some embodiments. Preferred embodiments provide CDRs shown in **FIG. 5** and **FIG. 6** and labeled accordingly on the figures.

The cDNAs bearing the nucleotide sequences coding the variable regions in immunoglobulin molecules can be cloned from hybridomas that produce the monoclonal antibody to PA, LF and/or EF of the tripartite anthrax exotoxin. To amplify the sequences, PCR can be performed. To identify active clones, ELISA can be used to determine binding to PA, LF and/or EF of the tripartite anthrax exotoxin. Further studies on affinities of an antibody that can bind to PA, LF and/or EF of the tripartite anthrax exotoxin can be determined with kinetic and thermodynamic studies using apparatus, such as BiaCore (Biacore, Piscataway, NJ) surface plasmon resonance apparatus for measuring binding affinity and binding kinetics. Thus, in one embodiment, specific cDNA sequences are provided.

In one embodiment, a monoclonal antibody that can block oligomerization of the PA component of anthrax exotoxin is provided. Accordingly, a monoclonal antibody of preferred embodiments can have preventive or therapeutic uses. A preferred monoclonal antibody can be used in a pharmaceutical composition as a vaccination for a mammal against anthrax or as a treatment for a mammal exposed to anthrax exotoxin. Accordingly, preferred embodiments provide methods of vaccinating a mammal against anthrax and/or treating a mammal exposed to anthrax.

A monoclonal antibody of several embodiments can be administered as a pharmaceutical composition. Thus, in one embodiment, the antibody can be administered by several different routes, including but not limited to: orally, parenterally and topically. The term "parenterally", as used herein, shall be given its ordinary meaning and shall also include subcutaneous, intravenous, intraarterial, injection or infusion techniques, without limitation. The term "topically", as used herein, shall be given its ordinary meaning and shall also encompasses administration rectally and by inhalation spray, as well as the more common routes of the skin and the mucous membranes of the mouth and nose. One skilled in the art will understand the appropriate dosage to be administered. Actual dosage levels of preferred antibody in a pharmaceutical composition may be varied so as to administer an amount of a preferred antibody that is effective to achieve the desired therapeutic response for a particular patient. The selected dosage level will depend upon the activity of the particular compound, the route of administration, the severity of the condition being treated, and the condition and prior medical history of the patient being treated. However, it is within the skill of the art to start doses of the compound at levels lower than required to achieve the desired therapeutic effect and to gradually increase the dosage until the desired effect is achieved. If desired, the effective daily dose may be divided into multiple doses for purposes of administration, e.g., two to four separate doses per day. It will be understood, however, that the specific dose level for any particular patient will depend upon a variety of factors including the body weight, general health, diet, time and route of administration, combination with other drugs and the severity of the particular disease being treated. According to several embodiments of the present invention, the pharmaceutical formulation can be in a variety of forms, including, but not limited to, injectable fluids, suppositories, powder, tablets, capsules, syrups, suspensions, liquids and elixirs.

Preferred embodiments of the present invention provide a kit for identifying the presence of anthrax exotoxin in a sample. In a preferred kit, there is a monoclonal antibody which specifically recognizes at least a portion of a component of an anthrax exotoxin. A sample is contacted with a monoclonal antibody which specifically recognizes at least a portion of a component of an anthrax exotoxin. If an anthrax exotoxin is present, then the binding of the anthrax exotoxin with the monoclonal antibody can be determined.

The disclosure below is of specific examples setting forth preferred methods for making compounds according to several embodiments of the present invention. These examples are not intended to limit the scope, but rather to exemplify preferred embodiments, For example, although the following examples describe the generation of antibodies to anthrax, antibodies to other antigens can also be made by following the examples set forth below. Various adaptations and modifications to adapt the protocols described herein will be understood by those skilled in the art.

### EXAMPLE 1

### INDIRECT ELISA

Flat bottom microtiter plates (Nunc F96 Maxisorp) were coated with 50µl of *Bacillus anthracis* Protective Antigen (PA) and Lethal Factor (LF)(List Biological Laboratories (City, State)) at a concentration of 1µg/mL in PBS overnight at 4°C. Plates were washed four times with PBS with Tween 20 at 0.1% and 50 µl of diluted sera was added to the wells for one hour at room temperature. Plates were washed as before and 50µl of secondary antibody, Goat anti-Human IgG, Fcγ specific, -HRP (Jackson Immuno Research 109-036-098) or Goat anti-Human IgM, Fc5µ specific, -HRP (Jackson Immuno Research 109-036-043) added and incubated for one hour at room temperature. After another wash step, 100µL of a substrate solution containing 0.4mg/mL OPD (O-phenlenediamine dihydrochloride) in citrate buffer (.025 M at pH 5.0) was added; after 15 minutes, 25µl of 3N HCl was added to stop the reaction and plates were then read on a Microplate reader (VersaMax, Molecular Devices, Sunnyvale, CA) at 490 nm.

### EXAMPLE 2

### RAW 264.7 CELL LINE IN VITRO BIOASSAY

The presence of neutralizing (protective) antibody to anthrax toxins PA and or LF in the antisera were determined using an in vitro protection bioassay with the mouse macrophage RAW 264.7 target cell line. Hanna, P. et al., Microbiology 90:10198 (1993). PA (100 ng/ml) and LF (50 ng/ml) were pre-incubated with the indicated dilutions of antiserum for 30 minutes at 37°C in a working volume of 100 ul of DMEM medium supplemented with 10% fetal calf serum, 2 mM L-glutamine, 100 IU/ml penicillin and 100µg/ml streptomycin. This 100µl volume was subsequently transferred into a 96 well flat bottom tissue culture plate containing 1x10⁴ RAW 264.7 cells/well in 100 µl of the same medium. The culture was incubated for 3 hours at 37°C. The wells were washed twice with media. The Residual attached cells were lysed and the released Lactate dehydrogenase (LDH) levels were measured using a CytoTox 96 kit (Cat#PAG1780 Promega, Madison, WI). Briefly, 10 µl Lysis Solution was added to 100 µl media per well and the mix Incubated 45 minutes in a humidified chamber at 37C, 5% CO2. An aliquot of the lysed material (50 µl) was transferred to a new plate and 50 µl assay buffer added. The plate was incubated for 30 minutes. Prior to adding 50 µl stop solution. The plates were read at 490 nm using a Tecan Spectra Fluor (Zurich, Switzerland) reader.

### EXAMPLE 3

### ENGRAFTMENT OF SCID MICE WITH HUMAN PBMC FROM ANTHRAX-VACCINATED DONORS

Peripheral blood mononuclear cells were enriched from whole blood of anthrax-vaccinated donors by density gradient using Histopaque, 1077-1 (Sigma, St. Louis, MO). One of skill in the art will understand that other types of cells can also be used in accordance with several embodiments of the present invention. Typically, one unit of blood from donors was obtained. Female SCID/bg 12 week old mice were each engrafted (via i.p. inoculation) with 2.5e7 isolated human PBMC. They were treated concomitantly i.p. with a volume of conditioned medium from the OKT8 mouse hybridoma grown in Ex-cell 620 hybridoma serum free medium (JRH, KS) and 2mM L-glutamine which contained 0.2 mg of the anti-CD8 antibody (used directly without further purification). The mice were immunized with a combination of PA and LF (i.p.) 2µg each adsorbed to Alum (Imject®, Pierce, Rockford, IL) and subsequently boosted (i.p.) on day 7, 19 and day 26. Mice were inoculated with 0.5 ml of EBV obtained from spent conditioned culture medium of the B95-8 marmoset cell line on day 7. Test bleeds were obtained from the orbital sinus on days 14 and 29. Two consecutive i.p. and iv boosts with PA and LF were administered (5 µg via each route on day 40 and 41, both in saline) prior to harvesting cells for fusion on day 42, also at which time an additional test bleed sample was obtained.

### EXAMPLE 4

### GENERATION OF HUMAN HYBRIDOMAS

Splenocytes, as well as large cell lymphomas (LCL) tumors were harvested on day 42 from those mice showing positive test bleeds in indirect ELISA. Human hybridomas were generated from these in separate fusions using a murine myeloma P3x63Ag8.653 with PEG-1500 (Sigma, St. Louis, MO) as described by Kearney JF, Radbruch A, Liesegang B, Rajewski K (1979, with the modification that the P3x63Ag8.653:lymphocyte ratio for fusion was between 1:3-1:5. A mouse myeloma cell line that has lost immunoglobulin expression, but permits the construction of antibody-secreting hybridoma cell lines. J Immunol 123: 1548-1558.

Although P3x63ag8.653 was used in this exemplary method, one skilled in the art will understand that several fusion partners can be used in accordance with various embodiments of the current invention, including, but not limited to, cells derived from the mouse myeloma MOPC21, triomas, etc. Double selection to select against the EBV-LCL and the unfused P3x63ag8.653 fusion partner was carried out using a combination of HAT selection and ouabain. A concentration of 8 µM ouabain (Sigma, St. Louis, MO) was used. One skilled in the art will appreciate that other poisons or toxins that interfere with the Na+/K+ ATPase can also be used in accordance with several embodiments of the present invention. In addition, one skilled in the art will understand that other selection methods can also be used.

### EXAMPLE 5

### TREATMENT AND SUBCLONING OF 21D9 HYBRIDOMA CELLS

16 days after fusion, hybridoma supernatants from 96 well plates were tested in indirect ELISA. Approximately 17 out of 1248 wells (13 plates) showed an initial positive ELISA signal on PA. All of them were chosen for further analysis and were subcloned at 5 cells/well on a feeder layer of irradiated NHLF (Cat# CC-2512, Cambrix, Baltimore, MD) in RPMI (Omega, San Diego, CA) supplemented with 10% FBS, 20% hybridoma cloning factor (IGEN, Gaithersboug, MD), 5ng/ml human IL6 (I-188, Leico), 1x HT (Sigma), 1x Vitamins (Omega), 1x Sodium pyruvate (Omega), 1x NEAA (Omega), 2x L-glutamine (Omega) and without antibiotics. The subcloning plates were tested in indirect ELISA after 10 days. Individual colonies from highly positive wells were hand-picked under a microscope using Pasteur pipets drawn out to fine points. After 2 weeks, individually picked clones were retested in indirect ELISA. Positive cells were recovered and the transcript mRNA encoding the immunoglobulins were reverse transcribed to form cDNA. Although the methodology for antibody 21 D9 is described herein, one of skill in the art will understand that the exemplary methodology described herein can also be used to make and test the other antibodies described and claimed herein.

### EXAMPLE 6

### VARIABLE REGION 21D9 IGG AND IGK cDNA CLONING AND EXPRESSION

Total RNA was prepared from specific ELISA positive hybridomas using RNeasy Mini Kit (Qiagen, Valencia, CA). Mixture of VH and VL cDNAs were synthesized and amplified in a same tube using One-Step RT-PCR Kit (Qiagen, Valencia, CA). Cycling parameters were 50 °C for 35min, 95 °C for 15min, 35 cycles of 94°C for 30 sec, 52 °C for 20 sec and 72 °C for 1 min 15sec, and 72 °C for 5min.

Primers were used for RT-PCR. These primers used for RT-PCR were:
**For VHγ**
   *Forward*
   a. CVH2 TGCCAGRTCACCTTGARGGAG
   b. CVH3 TGCSARGTGCAGCTGKTGGAG
   c. CVH4 TGCCAGSTGCAGCTRCAGSAG
   d. CVH6 TGCCAGGTACAGCTGCAGCAG
   e. CVH1257 TGCCAGGTGCAGCTGGTGSARTC
   *Reverse (located at 5' of CH1 region)*
   a. CγII GCCAGGGGGAAGACSGATG
**For VLκ**
   *Forward*
   a. VK1F GACATCCRGDTGACCCAGTCTCC
   b. VK36F GAAATTGTRWTGACRCAGTCTCC
   c. VK2346F GATRTTGTGMTGACBCAGWCTCC
   d.VK5F GAAACGACACTCACGCAGTCTC
   *Reverse (located in constant region)*
   a. Ck543 GTTTCTCGTAGTCTGCTTTGCTCA
**F**or **VLλ**
   *Forward*
   a. VL1 CAGTCTGTGYTGACGCAGCCGCC
   b. VL2 CAGTCTGYYCTGAYTCAGCCT
   c. VL3 TCCTATGAGCTGAYRCAGCYACC
   d. VL1459 CAGCCTGTGCTGACTCARYC
   e. VL78 CAGDCTGTGGTGACYCAGGAGCC
   f. VL6 AATTTTATGCTGACTCAGCCCC
   *Reverse (located in constant region)*
   a. CL2 AGCTCCTCAGAGGAGGGYGG

The RT-PCR was followed by nested PCR with High Fidelity Platinum PCR Mix (Invitrogen, Carlsbad, CA). A micro liter of RT-PCR products was used for VHγ, VLκ or VLλ specific cDNA amplification in the separate tube. At substantially the same time, restriction enzyme sites were introduced at both ends. Cycling parameters were 1 cycle of 94°C for 2 minutes, 6 °C for 30 seconds and 68°C for 45 seconds, 35 cycles of 94°C for 40 sseconds, 54 °C for 25 seconds and 68°C for 45 seconds, and 68°C for 5 minutes.

Each specific PCR product was separately purified, digested with restriction enzymes, and subcloned into appropriate mammalian full-length Ig expression vectors as described below.

### EXAMPLE 7

### SUBCLONING INTO VECTORS

Primers for nested PCR were used. These primers were as follows:
**For VH γ**
   *Forward (adding BsrGI site at 5' end)*
   a. BsrGIVHF2 AAAATGTACAGTGCCAGRTCACCTTGARGGAG
   b. BsrGIVHF3 AAAATGTACAGTGCSARGTGCAGCTGKTGGAG
   c. BsrGIVHF4 AAAATGTACAGTGCCAGSTGCAGCTRCAGSAG
   d. BsrGIVHF6 AAAATGTACAGTGCCAGGTACAGCTGCAGCAG
   e. BsrGIVHF1257 AAAATGTACAGTGCCAGGTGCAGCTGGTGSARTC
   *Reverse (including native Apal site)*
   a. C γ ER GACSGATGGGCCCTTGGTGGA

VHyPCR products are digested with BsrG I and Apa I and ligated into pEEG1.1 vector that is linearlized by Spl I and Apa, I double digestion.
**For VLκ**
   *Forward (adding AgeI site, Cys and Asp at 5'end)*
   a. AgeIVK1F TTTTACCGGTGTGACATCCRGDTGACCCAGTCTCC
   b. AgeIVK36F TTTTACCGGTGTGAAATTGTRWTGACRCAGTCTCC
   c. AgeIVK2346F TTTTACCGGTGTGATRTTGTGMTGACBCAGWCTCC
   d. AgeIVK5F TTTTACCGGTGTGAAACGACACTCACGCAGTCTC
   *Reverse (adding SplI site, located between FR4 and 5' of constsnt region)*
   a. SplKFR4R12 TTTCGTACGTTTGAYYTCCASCTTGGTCCCYTG
   b. SplKFR4R3 TTTCGTACGTTTSAKATCCACTTTGGTCCCAGG
   c. SplKFR4R4 TTTCGTACGTTTGATCTCCACCTTGGTCCCTCC
   d. SplKFR4R5 TTTCGTACGTTTAATCTCCAGTCGTGTCCCTTG

VLκ PCR products are digested with Age I and Spl I and ligated into pEEK1.1 vector linearlized by Xma I and Spl I double digestion.
**For VLλ**
   *Forward (adding ApaI site at 5' end)*
   a. ApaIVL1 ATATGGGCCCAGTCTGTGYTGACGCAGCCGCC
   b. ApaIVL2 ATATGGGCCCAGTCTGYYCTGAYTCAGCCT
   c. ApaIVL3 ATATGGGCCCAGTATGAGCTGAYRCAGCYACC
   d. ApaIVL1459 ATATGGGCCCAGCCTGTGCTGACTCARYC
   e. ApaIVL78 ATATGGGCCCAGDCTGTGGTGACYCAGGAGCC
   f. ApaIVL6 ATATGGGCCCAGTTTTATGCTGACTCAGCCCC
   *Reverse (adding Avr II site, located between FR4 and 5' of constant region)*
   a. AvrIIVL1IR TTTCCTAGGACGGTGACCTTGGTCCCAGT
   b. AvrIIVL237IR TTTCCTAGGACGGTCAGCTTGGTSCCTCCKCCG
   c. AvrIIVL6IR TTTCCTAGGACGGTCACCTTGGTGCCACT
   d. AvrIIVLmixIR TTTCCTAGGACGGTCARCTKGGTBCCTCC

VLλPCR products are digested with Apa I and Avr II and ligated into pEELg vector linearlized by Apa I and Avr II double digestion. The positive clones were identified after transient co-transfection by determining expression in the supernatants by indirect ELISA on PA coated plates. CHO K1 cells were transfected with different combinations of IgG and IgK cDNAs using Lipofectamine-2000 (Invitrogen, Carlsbad, CA). The supernatants were harvested about 48 hours to about 72 hours after transfection. Multiple positive clones were sequenced with the ABI 3700 automatic sequencer (Applied Biosystems, Foster City, CA) and analyzed with Sequencher v4.1.4 software (Gene Codes, Ann Arbor, MI).

### EXAMPLE 8

### STABLE CELL LINE ESTABLISHMENT

Ig heavy chain or light chain expression vector were double digested with Not I and Sal I, and then both fragments were ligated to form a double gene expression vector. CHO-K1 cells in 6 well-plate were transfected with the double gene expression vector using Lipofectamine 2000 (Invitrogen, Carlsbad, CA). After 24 hrs transfection cells were transferred to 10 cm dish with selection medium (D.MEM supplemented with 10% dialyzed FBS, 50 µM L-methionine sulphoximine (MSX), penicillin/streptomycin, GS supplement). Two weeks later MSX resistant transfectants were isolated and expanded. Anti-PA antibody high producing clones were selected by measuring the supernatant with PA specific ELISA assay. MSX concentration was increased from 50 µM to 100 µM to enhance the antibody productivity.

### EXAMPLE 9

### SERUM FREE ADAPTATION PROCEDURE

Cells were thawed out from liquid nitrogen storage, the cells were in 10% FBS in ExCell 302 Serum Free Medium (JRH, cat.# 14312-1000M) with Ix GS (JRH, cat.# 58672-100M) and 25-100mM L-Methionine Sulphoximine(Sigma, cat.# M5379). Cells were treated with trypsin (OMEGA, cat.# TE-91) and split by 1:5. The culture medium was switched to 5% FBS containing media and the cells cultured 2 days. When the cells adapted to growing in 5% FBS containing media, the media was changed to 100% of serum free media + 2.5% di FBS for 1-2 days, then to 100% of serum free media. At this point, the cells were in suspension. The cells were expanded from to Integra flasks for small scale production in serum free media. Purification was carried out by filtering the spent culture media through a 0.2µ filter and then loaded directly to a HiTrap Protein A column (Pharmacia), followed by washing with 20 mM Sodium phosphate pH 7.4, and the antibody eluted with 0.1M glycine HCl pH3.4 and immediately neutralized with 1/10 volume of 1M Tris-HCl pH 8.0. The fraction protein content was determined by absorbance at 280 nm, the fractions containing antibody were pooled and dialyzed against phosphate buffered saline pH 7.4 (2x 500 volumes) and filter sterilized through 0.2 µ filter. The antibody was further characterized by SDS-PAGE and the purity exceeded 95%.

### EXAMPLE 10

### AFFINITY DETERMINATIONS

Affinity constants were determined using the principal of surface plasmon resonance (SPR) with a Biacore 3000 (Biacore Inc.). A Biacore CM5 chip was used with affinity purified goat anti-human IgG+A+M (Jackson ImmunoResearch) conjugated to two flowcells of the CM5 chip according to manufacturer's instructions. An optimal concentration of an antibody preparation is first introduced into one of the two flowcells, and is captured by the anti-human Ig. Next, a defined concentration of antigen is introduced into both flowcells for a defined period of time, using the flowcell without antibody as a reference signal. As antigen binds to the captured antibody of interest, there is a change in the SPR signal, which is proportional to the amount of antigen bound. After a defined period of time, antigen solution is replaced with buffer, and dissociation of the antigen from the antibody is then measured, again by the SPR signal. Curve-fitting software provided by Biacore generates estimates of the association and dissociation rates, and affinities.

The results from this study are summarized in Table 1, below. The equilibrium dissociation constant (K_{d}) for recombinant form of the 21D9 MAb was determined by BiaCore analyses. The rate constants kₒₙ and k_{off} were evaluated directly from the sensogram in the BiaCore analysis and the K_{d} was deduced.

**Table 1. Affinity determination of antibody 21 D9 and other antibodies on PA (83 Kd)**

| **Antibody** | **Dissociation Constant (K_{D}) M** | **Association Rate (kₒₙ)** | **Dissociation Rate (k_{off})** |
|---|---|---|---|
| **AVP-21 D9** | **8.21 x 10⁻¹¹** | **1.80 x 10⁵** | **1.48 x 10⁻⁵** |
| **AVP-1C6** | **7.11 x 10⁻¹⁰** | **1.85 x 10⁵** | **1.31 x 10⁻⁴** |
| **AVP-4H7** | **1.41 x 10⁻¹⁰** | **1.74 x 10⁵** | **2.45 x 10⁻⁵** |
| **AVP-22G12** | **5.12 x 10⁻¹⁰** | **1.01 x 10⁵** | **5.17 x 10⁻⁵** |

### EXAMPLE 11

### HUMAN IGG QUANTIFICATION BY IMMUNOENZYMETRIC ASSAY

Flat bottom microtiter plates (Nunc F96 Maxisorp) were coated overnight at 4°C with 50µl of Goat anti-Human IgG, Fcγ specific, (cat# 109-005-098, Jackson Immuno Research, West Grove, Pennsylvania) at 1 µg/mL in PBS. Plates were washed four times with PBS-0.1% Tween 20. Meanwhile, in a separate preparation plate, dilutions of standards (in duplo) and unknowns were prepared in 100 µl volume of PBS with 1 mg/ml BSA. A purified monoclonal human IgG1K myeloma protein (cat# I-5154 Sigma, St. Louis, MO) was used as the standard and a different IgG1K myeloma protein (Athens Research, Athens, Georgia) served as an internal calibrator for comparison. Diluted test samples (50 µl) were transferred to the wells of the assay plate and incubated for one hour at room temperature. Plates were washed as before and 50 µl of the detecting antibody (1:4000 in PBS with 1mg/ml BSA.) Goat anti-Human Kappa-HRP (Cat. # 2060-05 Southern Biotechnology Associates, Inc., Birmingham, Alabama) was added and incubated for one hour at room temperature. After another wash step, 100 µL of a substrate solution containing 0.4 mg/mL OPD (O-phenlenediamine dihydrochloride) in citrate buffer (.025 M at pH 5.0) was added. Following a 15 minute substrate incubation, 25µl of 3N HCl stop solution was added and plates were read on a Microplate reader (VersaMax, Molecular Devices, Sunnyvale, Ca) at 490 nm. Unknowns were interpolated from standard curve values using SoftMaxPro v 4.0 software (Sunnyvale, CA).

### EXAMPLE 12

### RESULTS

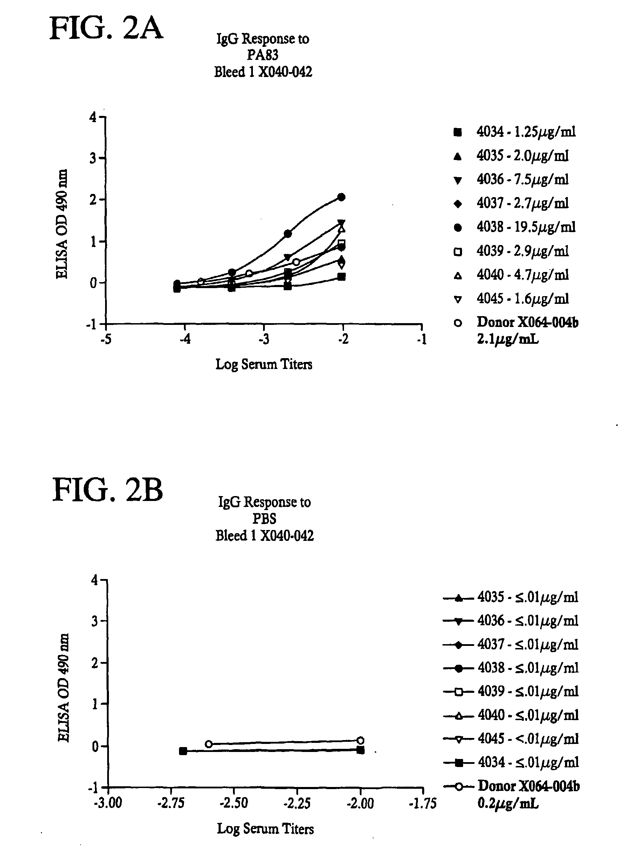

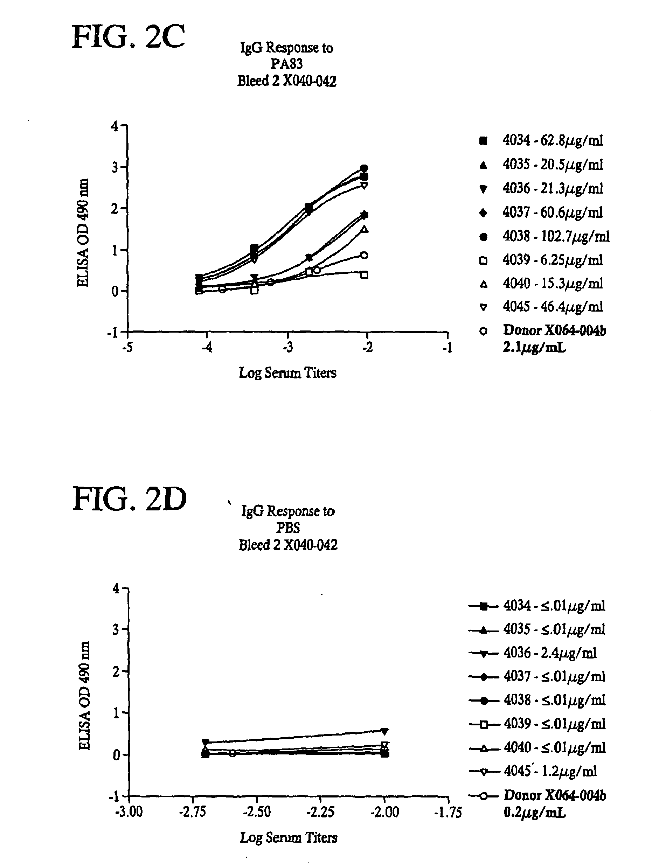

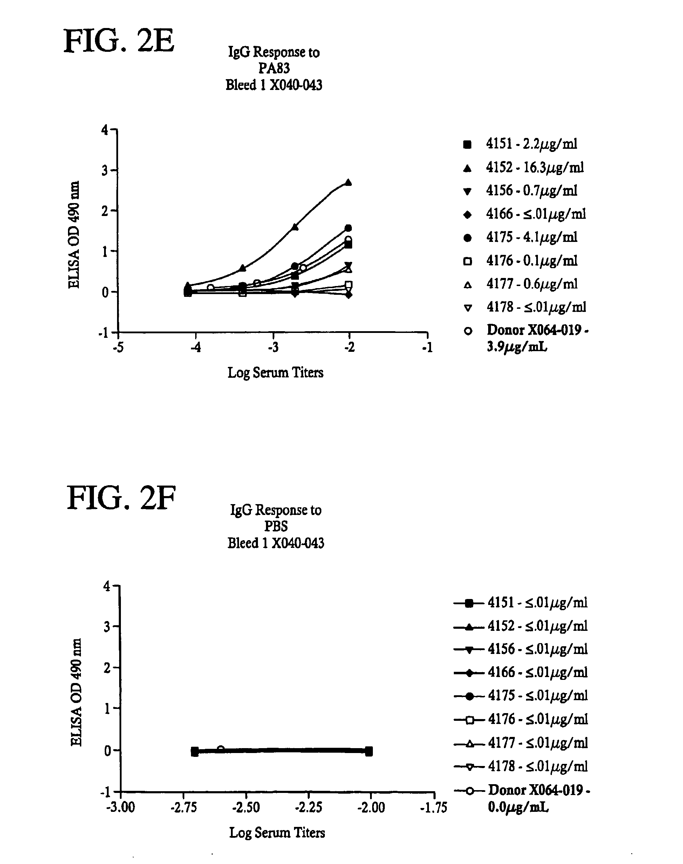

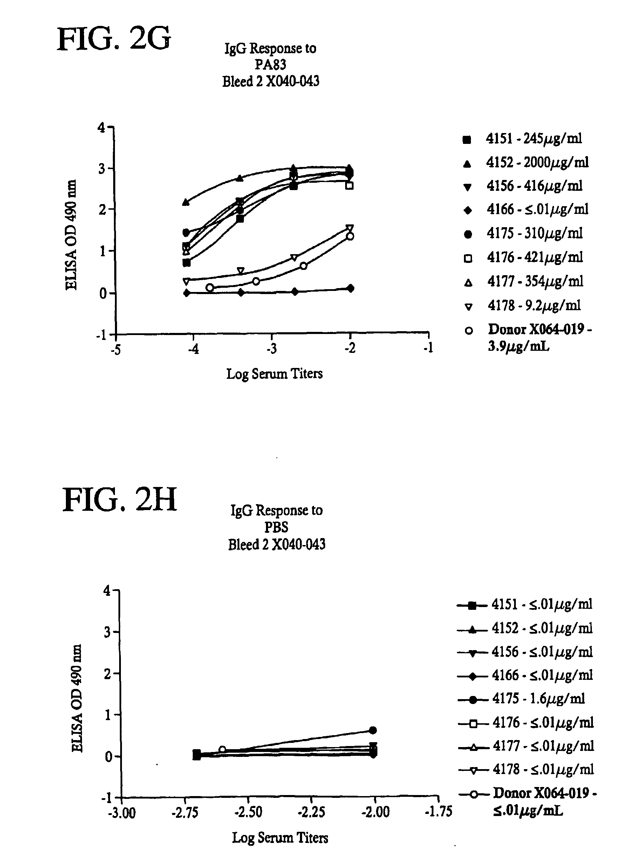

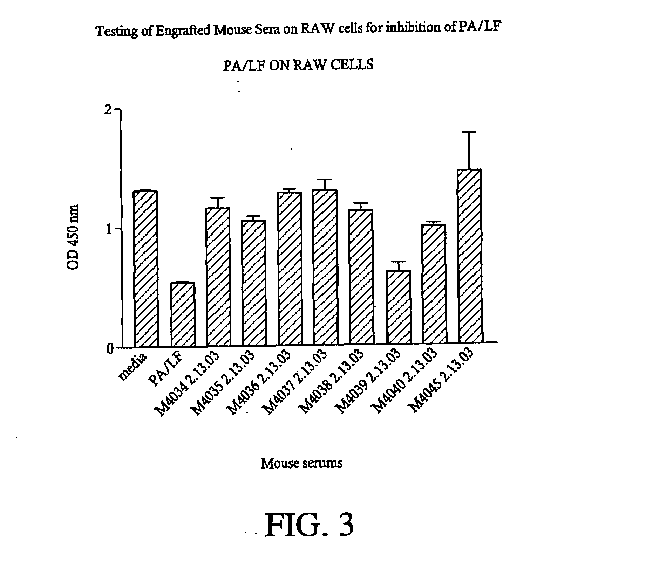

Testbleeds from mice engrafted with human PBMC from an anthrax-vaccinated donor and further boosted via immunization in vivo were obtained. **FIG. 2A-H** shows comparison results of the anti-anthrax toxin levels in the donor plasma as compared to the sera of engrafted mice. **FIG. 2A-H** shows that the mouse sera level of functional immunoreactive (Indirect ELISA) antibody is considerably greater higher than that observed in the donor. A range of levels of immunoreactive antibody was observed in the engrafted mice. Test bleeds from engrafted mice were also evaluated for the presence of anti-PA/LF protective antibody in the mouse macrophage RAW cell bioassay (Figure 3). In this bioassay, the translocation of PA/LF complex into the cell triggers signal transduction events (MAPKK mediated) that lead to cell death, and a lower bioassay signal. The presence of protective antibody reverses this. The original donor plasma did not appear to contain detectable levels of protective antibody (even when tested at a lower dilution) in comparison with the engrafted mice. Both the increase in immunoreactive (ELISA) antibody and the appearance of seroprotection in the engrafted mice show the amplification of a seroprotective immune response to anthrax toxin elicited by repeated immunization of human PBMC-engrafted SCID mice. In one embodiment, the presence of appropriate seropositivity is one preferred criterion for selecting appropriate animals for fusion to generate human hybridomas.

A series of 14 individual fusions was carried out with cells obtained from various compartments (either peritoneal wash (PW), spleen (SP), or LCL tumors (TU) within the peritoneal cavity) of the engrafted mice. In several fusions, the cells were pooled from several engrafted mice determined to be producing specific anti-anthrax toxin antisera by Indirect ELISA and RAW Cell bioassay prior to fusion. A summary of the fusion results is shown in Table 2.

**Table 2 Origin of resulting hybridomas obtained in IJ-8 Anti-Anthrax Toxin Study**

| **Plate** # | **Mouse #** | **Cell Sources** | **Partner** | **Positive Wells** | **Subcloned** |
|---|---|---|---|---|---|
| **1** | 4034/35/37/38/40/ 41 | PW | P3X | **0** | - |
| **2** | 4034/35/37/38/40/ 41 | PW | P3X | **0** | - |
| **3** | 4034/35/37/38/40/ 41 | PW | P3X | **0** | - |
| **4** | 4037/38 | SP | P3X | **0** | - |
| **5** | 4034/45 | SP | P3X | **6** | + |
| **6** | 4034/45 | SP | P3X | **10** | + |
| **7** | 4035/40 | SP | P3X | **0** | - |
| **8** | 4035/40 | SP | P3X | **0** | - |
| **9** | 4035/40 | SP | P3X | **0** | - |
| **10** | 4035/40 | SP | P3X | **0** | - |
| **11** | 4035/40 | SP | P3X | **1** | + |
| **12** | 4035/38/40 | TU | P3X | **0** | - |
| **13** | 4035/38/40 | TU | P3X | **0** | - |
| **14** | 4034/45 | TU | P3X | **0** | - |

Hybridomas were initially selected based on ability to bind to PA (83kD) protein adsorbed to polystyrene microtiter plate wells in an indirect ELISA. A wide range of values for the relative amount of specific anti-PA antibody in the supernatants was observed. In parallel, each of the supernatants was tested individually at a in the Anthrax toxin protection RAW cell bioassay.

A dose-response curve of hybridoma-derived 21D9 in the RAW cell bioassay was used to evaluate the effective in vitro IC₅₀ protective concentration using a cocktail of the PA (83kD) and LF toxins. An antibody IC₅₀ of 0.21nM was observed **(****FIG. 5****).**

The 21D9 antibody was found to bind to the intact (83kD) form as well as the cleaved (63kD) form of the PA toxin, but to a lesser degree the heptamer using BiaCore (Pharmacia, Peapack, NJ). Additionally, there was no evidence that the antibody was able to inhibit LF binding to PA (63 kD) heptamer as determined by sequential incubations in the BiaCore. This finding potentially implicates the domain 2 on the PA toxin as the epitope blocked by this antibody.

The nucleotide sequences of the 21D9 MAb heavy and light chains variable regions were determined **(****FIG. 5** and **FIG. 6****).**

The alignment of variable regions using V BASE DNAPLOT software (18) showed that 21D9 heavy chain used VH gene from VH3 family (3-43 locus), D region segment 6-19 (in first reading frame) with N region addition and JH4b. 21D9 light chain was from VKI family (L12 locus), and used JK1 region segment. The number of mutations from most closely related germline were 26 (heavy chain) and 14 (light chain). Comparisons with germline V genes suggest that the 21D9 V regions had undergone extensive somatic mutations characteristic of an Ag-driven immune response.

### EXAMPLE 11

### HUMAN MONOCLONAL ANTIBODIES FROM IMMUNIZED DONORS ARE PROTECTIVE AGAINST ANTHRAX LETHAL TOXIN IN VIVO.

Anthrax exotoxins, the dominant virulence factors produced by *Bacillus anthracis* are a tripartite combination of protective antigen (PA), lethal factor (LF) and edema factor (EF). These toxins are thought to have a critical role in anthrax pathogenesis; initially to impair the immune system, permitting the anthrax bacterium to evade immune surveillance to disseminate and reach high concentrations; and later in the infection the toxins may contribute directly to death in the host animals including humans. Antibodies that neutralize the PA component of the exotoxin could provide an effective protection from anthrax toxin exposure, early and potentially late in the infection. In one embodiment, the generation of a panel of very potent fully human anti-PA neutralizing antibodies derived from PBMCs obtained from vaccinated donors is provided.

The antibodies were generated through the combined use *of in vivo* immunization of SCID mice reconstituted with human PBMC (U.S. Patent Nos. 5,476,996 5,698,767 5,811,524, 5,958,765, 6,413,771, 6,537,809), subsequent recovery of human B cells expressing anti-PA antibodies and immortalization via cell fusion with the mouse myeloma cells. Human immunoglobulin cDNAs were isolated and subcloned into the mammalian expression vector. Recombinant antibodies were first screened by *in vitro* neutralization assay using RAW264.7 mouse macrophage cell line. Furthermore, selected antibodies were evaluated for neutralization of lethal toxin *in vivo* in the Fisher 344 rat bolus toxin challenge model.

Analysis of the variable regions indicated that antibodies recovered from SCID mice were diverse and hyper-mutated. Among these antibodies, a single IV administration of AVP-21D9 or AVP-22G12 was found to confer full protection with only 0.5x (AVP-21D9) or 1x (AVP-22G12) molar excess relative to PA in the rat toxin challenge prophylaxis model. Aglycosylated PA neutralizing antibodies also protected rats from lethal toxin challenge. Although not wishing to be bound by the following theory, it is believed that the PA toxin neutralizing *activity in vivo* is not depended on Fc mediated effector functions.

In one embodiment, these potent fully human anti-PA toxin-neutralizing antibodies generated may be used *for in vivo* human use for prophylaxis and/or treatment against Anthrax Class A bioterrorism toxins.

### EXAMPLE 12

### CHARACTERIZATION OF A PANEL OF POTENT ANTHRAX TOXIN NEUTRALIZING HUMAN MONOCLONAL ANTIBODIES FROM IMMUNIZED DONORS

In one embodiment, antibodies that bind to the PA component of the tripartite anthrax-toxin and which provide protection as single agents are provided. In one embodiment, antibody 21D9 is provided. In another embodiment, antibody 22G12 is provided. In a further embodiment, antibody 1C6 is provided. In one embodiment, these antibodies are used as single agent in preventing and/or treating anthrax infection. In other embodiments, combination of two or more of these antibodies are used to treat mammals who have been exposed to aerosolized *Bacillus anthracis* spores, or exposed to other forms of anthrax.

In one embodiment, antibodies that bind to PA with a range of high affinities, from about 82 pM to about 700 pM, as determined by surface plasmon resonance (BiaCore 3000), is provided. Experimental data showed that antibodies 1C6, 21D9 and 22G12, that recognize unique non-competing sites and also do not appear to interfere with PA recognition of soluble TEM-8 are provided. The biological efficacy of these three antibodies were determined in an in vitro anthrax lethal toxin neutralization assay. All three antibodies protected RAW 264.7 cell from toxin induced cell death and provided 50% neutralization at sub-equimolar ratio of antibody to toxin.

While a number of preferred embodiments of the invention and variations thereof have been described in detail, other modifications and methods of use will be readily apparent to those of skill in the art. Accordingly, it should be understood that various applications, modifications and substitutions may be made of equivalents without departing from the spirit of the invention or the scope of the claims.

### Embodiments

Embodiment 1. A fully human monoclonal antibody or fragment thereof which specifically recognizes at least a portion of an anthrax exotoxin.
Embodiment 2. The fully human monoclonal antibody or fragment thereof of embodiment 1 , wherein said portion of an anthrax exotoxin is selected from the group consisting of protective antigen (PA), lethal factor (LF) and edema factor (EF).
Embodiment 3. A fully human immunoglobulin or fragment thereof, that recognizes at least a portion of an anthrax exotoxin, wherein said immunoglobulin or fragment thereof comprises an immunoglobulin heavy chain comprising the amino acid sequence shown in FIG. 5.
Embodiment 4. The immunoglobulin of embodiment 3, wherein said immunoglobulin or fragment thereof comprises an immunoglobulin heavy chain comprising CDR1, CDR2, and CDR3; wherein
   said CDR1 is comprised of the amino acid sequence, as shown in FIG. 5;
   said CDR2 is comprised of the amino acid sequence, as shown in FIG. 5; and
   said CDR3 is comprised of the amino acid sequence, as shown in FIG. 5.
Embodiment 5. The immunoglobulin of embodiment 3, wherein said immunoglobulin or fragment thereof is encoded by the nucleotide sequence shown in FIG. 5.
Embodiment 6. The immunoglobulin of Embodiment 4, wherein said CDR1, CDR2, and CDR3 are encoded by the nucleotide sequences, as shown respectively in FIG. 5.
Embodiment 7. The immunoglobulin of embodiment 3, wherein said portion of an anthrax exotoxin is selected from the group consisting of PA, LF and EF.
Embodiment 8. A fully human immunoglobulin or fragment thereof, that recognizes at least a portion of an anthrax exotoxin, wherein said immunoglobulin or fragment thereof comprises an immunoglobulin light chain comprising the amino acid sequence shown in FIG. 6.
9. The immunoglobulin of embodiment 8, wherein said immunoglobulin or fragment thereof comprises an immunoglobulin light chain comprising CDR1, CDR2, and CDR3; and,
   said CDR1 is comprised of the amino acid sequence, as shown in FIG. 6;
   said CDR2 is comprised of the amino acid sequence, as shown in FIG. 6; and
   said CDR3 is comprised of the amino acid sequence, as shown in FIG. 6.
Embodiment 10. The immunoglobulin of embodiment 8, wherein said immunoglobulin or fragment thereof is encoded by the nucleotide sequence shown in FIG. 6.
Embodiment 11. The immunoglobulin of embodiment 9, wherein said CDR1, CDR2, and CDR3 are encoded by the nucleotide sequences, as shown respectively in FIG. 6.
Embodiment 12. The immunoglobulin of embodiment 8, wherein said portion of an anthrax exotoxin is selected from the group consisting of PA, LF and EF.
Embodiment 13. The fully human immunoglobulin or fragment thereof of embodiment 1, which is a single chain that recognizes at least a portion of an anthrax exotoxin.
Embodiment 14. A fully human immunoglobulin or fragment thereof, that recognizes at least a portion of an anthrax exotoxin, wherein said immunoglobulin or fragment thereof comprises an immunoglobulin heavy chain comprising at least one complementary determining region selected from the group consisting of CDR1, CDR2 and CDR3; and
   said CDRI is comprised of the amino acid sequence, as shown in FIG. 5;
   said CDR2 is comprised of the amino acid sequence, as shown in FIG. 5; and
   said CDR3 is comprised of the amino acid sequence, as shown in FIG. 5.
Embodiment 15. A fully human immunoglobulin or fragment thereof, that recognizes at least a portion of an anthrax exotoxin, wherein said immunoglobulin or fragment thereof comprises an immunoglobulin light chain comprising at least one complementary determining region selected from the group consisting of CDR1, CDR2 and CDR3; and
   said CDR1 is comprised of the amino acid sequence, as shown in FIG. 6;
   said CDR2 is comprised of the amino acid sequence, as shown in FIG. 6; and
   said CDR3 is comprised of the amino acid sequence, as shown in FIG. 6.
Embodiment 16. A fully human immunoglobulin or fragment thereof, that recognizes at least a portion of an anthrax exotoxin, wherein said immunoglobulin or fragment thereof comprises an immunoglobulin heavy chain comprising the amino acid sequence shown in FIG. 8.
Embodiment 17. A fully human immunoglobulin or fragment thereof, that recognizes at least a portion of an anthrax exotoxin, wherein said immunoglobulin or fragment thereof comprises an immunoglobulin light chain comprising the amino acid sequence shown in FIG. 8.
Embodiment 18. A fully human immunoglobulin or fragment thereof, that recognizes at least a portion of an anthrax exotoxin, wherein said immunoglobulin or fragment thereof comprises an immunoglobulin heavy chain comprising the amino acid sequence shown in FIG. 9.
Embodiment 19. A fully human immunoglobulin or fragment thereof, that recognizes at least a portion of an anthrax exotoxin, wherein said immunoglobulin or fragment thereof comprises an immunoglobulin light chain comprising the amino acid sequence shown in FIG. 9.
Embodiment 20. A fully human immunoglobulin or fragment thereof, that recognizes at least a portion of an anthrax exotoxin, wherein said immunoglobulin or fragment thereof comprises an immunoglobulin heavy chain comprising the amino acid sequence shown in FIG. 10.
Embodiment 21. A fully human immunoglobulin or fragment thereof, that recognizes at least a portion of an anthrax exotoxin, wherein said immunoglobulin or fragment thereof comprises an immunoglobulin light chain comprising the amino acid sequence shown in FIG. 10.
Embodiment 22. A nucleotide sequence, shown in FIG. 5, encoding a heavy chain variable region of a fully human immunoglobulin or fragment thereof, that recognizes at least a portion of an anthrax exotoxin.
Embodiment 23. A nucleotide sequence, shown in FIG. 6, encoding a light chain variable region of a fully human immunoglobulin or fragment thereof, that recognizes at least a portion of an anthrax exotoxin.
Embodiment 24. A nucleotide sequence, shown in FIG. 8, encoding a heavy chain variable region of a fully human immunoglobulin or fragment thereof, that recognizes at least a portion of an. anthrax exotoxin.
Embodiment 25. A nucleotide sequence, shown in FIG. 8, encoding a light chain variable region of a fully human immunoglobulin or fragment thereof, that recognizes at least a portion of an anthrax exotoxin.
Embodiment 26. A nucleotide sequence, shown in FIG. 9, encoding a heavy chain variable region of a fully human immunoglobulin or fragment thereof, that recognizes at least a portion of an anthrax exotoxin.
Embodiment 27. A nucleotide sequence, shown in FIG. 9, encoding a light chain variable region of a fully human immunoglobulin or fragment thereof, that recognizes at least a portion of an anthrax exotoxin.
Embodiment 28. A nucleotide sequence, shown in FIG. 10, encoding a heavy chain variable region of a fully human immunoglobulin or fragment thereof, that recognizes at least a portion of an anthrax exotoxin.
Embodiment 29. A nucleotide sequence, shown in FIG. 10, encoding a light chain variable region of a fully human immunoglobulin or fragment thereof, that recognizes at least a portion of an anthrax exotoxin.
Embodiment 30. A method of generating a fully human monoclonal antibody which specifically recognizes at least a portion of an anthrax exotoxin comprising:
   administering peripheral blood mononuclear cells from one or more human donors exposed to anthrax to an immuno-compromised animal;
   isolating at least one lymphocytic cell from said animal; and
   fusing said at least one lymphocytic cell with a hybridoma fusion partner, thereby generating a fully human monoclonal antibody.
Embodiment 31. The method of embodiment 30, further comprising screening the generated antibodies.
Embodiment 32. The method of embodiment 30, further comprising transforming at least a portion of said lymphocytic cells with EBV.
Embodiment 33. The method of embodiment 30, further comprising characterizing the animal's immune response using a test bleed.
Embodiment 34. The method of embodiment 30, further comprising administering one or more booster injections of anthrax antigen to the animal.
Embodiment 35. The method of embodiment 30, further comprising administering one or more injections of anti-CD8 to the animal.
Embodiment 36. The method of embodiment 30, further comprising using a double selection method to select against undesirable cells.
Embodiment 37. The method of embodiment 36, wherein the double selection method comprises using HAT selection.
Embodiment 38. The method of embodiment 36, wherein the double selection method comprises using ouabain.
Embodiment 39. The method of embodiment 30, wherein the human donor is an anthrax-vaccinated donor.
Embodiment 40. The method of embodiment 30, wherein the human donor has been inadvertently exposed to anthrax.
Embodiment 41. The method of embodiment 30, wherein the animal is a SCID mouse.
Embodiment 42. The method of embodiment 30, wherein the hybridoma fusion partner is derived from a mouse myeloma MOPC21.
Embodiment 43. The method of embodiment 30, wherein the hybridoma fusion partner is a myeloma.
Embodiment 44. The method of embodiment 30, wherein the hybridoma fusion partner is P3x63Ag8.653.
Embodiment 45. The method of embodiment 30, wherein said portion of an anthrax exotoxin is selected from the group consisting of PA, LF and EF.
Embodiment 46. A method for inhibiting the assembly of the protective antigen (PA) of an anthrax exotoxin on receptors in a human, the method comprising administering to such human the antibody of any of embodiments 1-21.
Embodiment 47. A pharmaceutical composition for vaccinating a mammal against anthrax, comprising the fully human monoclonal antibody of any of embodiments 1-21.
Embodiment 48. A pharmaceutical composition for treating a mammal exposed to an anthrax exotoxin, comprising the fully human monoclonal antibody of any of embodiments 1-21.
Embodiment 49. A method of vaccinating a mammal against anthrax, comprising:
   administering an immunizing dose of the fully human monoclonal antibody of any of embodiments 1-21 to said mammal.
Embodiment 50. A method of treating a mammal exposed to anthrax, comprising:
   administering a therapeutic dose of the fully human monoclonal antibody of any of embodiments 1-21 to said mammal.
Embodiment 51. A method of identifying the presence of anthrax exotoxin in a sample, comprising:
   contacting at least a portion of said sample with the fully human monoclonal antibody of any of embodiments 1-21 ; and
   determining binding of anthrax exotoxin with said antibody, wherein said binding is an indicator of the presence anthrax in said sample.
Embodiment 52. A kit to identify the presence of anthrax exotoxin in a sample, comprising:
   the fully human monoclonal antibody of any of embodiments 1-21; and
   an assay system to determine the binding of anthrax exotoxin with said antibody, wherein said binding is an indicator of the presence anthrax in said sample.
Embodiment 53. The immunoglobin of embodiments 1-21, wherein the anthrax exotoxin is tripartite.
Embodiment 54. The immunoglobin of embodiments 1-21, wherein the anthrax exotoxin is naturally-occurring or synthetic.

<110> Avanir Pharmaceuticals, Inc.

<120> NEUTRALIZING HUMAN ANTIBODIES TO ANTHRAX TOXIN

<130> GHS/P506926EP

<140> EP03816309.3
<141> 14-11-2003

<150> PCT/US2003/36555
<151> 14-11-2003

<160> 58

<170> FastSEQ for Windows Version 4.0

<210> 1
<211> 363
<212> DNA
<213> Homo sapiens

<400> 1
caggtacagc tgcagcagtc tgggggagcc gtggtgcagc ctggggggtc cctcagactc 60
tcctgtgcag cctctggatt cacgcttgat gattatgcca tgcactgggt ccgacaagtt 120
tcggggaagg gtctggagtg ggtctgcctt gtcagttggg atggtcatgc cacccactat 180
gcagactctg tgaagggtcg attcaccatc tccagagaca acagcagaaa ctccctgttt 240
ctgcaaatgg acggtctgag acctgaggac accgccttgt attactgtgt aaaagcattt 300
agtagtggct ggtctgatgc ttttcacttc tggggccagg gaaccctggt caccgtctcc 360
tca 363

<210> 2
<211> 121
<212> PRT
<213> Homo sapiens

<400> 2
Gln Val Gln Leu Gln Gln Ser Gly Gly Ala Val Val Gln Pro Gly Gly
1               5                   10                  15
Ser Leu Arg Leu Ser Cys Ala Ala Ser Gly Phe Thr Leu Asp Asp Tyr
            20                  25                  30
Ala Met His Trp Val Arg Gln Val Ser Gly Lys Gly Leu Glu Trp Val
        35                  40                  45
Cys Leu Val Ser Trp Asp Gly His Ala Thr His Tyr Ala Asp Ser Val
    50                  55                  60
Lys Gly Arg Phe Thr Ile Ser Arg Asp Asn Ser Arg Asn Ser Leu Phe
65                  70                  75                  80
Leu Gln Met Asp Gly Leu Arg Pro Glu Asp Thr Ala Leu Tyr Tyr Cys
                85                  90                  95
Val Lys Ala Phe Ser Ser Gly Trp Ser Asp Ala Phe His Phe Trp Gly
            100                 105                 110
Gln Gly Thr Leu Val Thr Val Ser Ser
        115                 120

<210> 3
<211> 318
<212> DNA
<213> Homo sapiens
<400> 3
gaaattgtgt tgacvcagtc tccttccacc ctgtctgcgt ctgtagggga cagagtcatt 60
atcacttgcc gggccagtca gaggattcgt aacgagttgg cctggtatca gcagaaacca 120
gggaaagccc ctaaagtcct gatctataag gcgtctactt tagaaagtgg ggtcccatca 180
aggttcagcg gcagtggatc tgggacagaa ttcactctca ccatcagcag cctgcagcct 240
gatgattttg caacttatta ctgccaacaa tatagtggtt tgtggacgtt cggccagggg 300
accaagctgg aaatcaaa 318

<210> 4
<211> 106
<212> PRT
<213> Homo sapiens

<400> 4
Glu Ile Val Leu Thr Gln Ser Pro Ser Thr Leu Ser Ala Ser Val Gly
1               5                   10                  15
Asp Arg Val Ile Ile Thr Cys Arg Ala Ser Gln Arg Ile Arg Asn Glu

                20                  25                  30
Leu Ala Trp Tyr Gln Gln Lys Pro Gly Lys Ala Pro Lys Val Leu Ile
        35                  40                  45
Tyr Lys Ala Ser Thr Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ser Gly Leu Trp Thr
                85                  90                  95
Phe Gly Gln Gly Thr Lys Leu Glu Ile Lys
            100                 105

<210> 5
<211> 375
<212> DNA
<213> Homo sapiens

<400> 5
caggtacagc tgcagcagtc tgggggaggc ttggtccagc ctggggggtc cctcaaactc 60
tcctgtgcag cctctgggtt caccttcagt gactctgcta ttcactgggt ccgccaggct 120
tccgggaaag ggctggagtg ggttggccgt attagaagca aagctaacgg ttacgcgaca 180
gcatatactg cgtcggtgaa aggcaggttc accatctcca gagatgattc actgaacacg 240
gcgtatctgc aaatgaacag cctgaaaacc gaggacacgg ccgtgtatta ctgcactaga 300
cacgatagca ccacctggtt cttgagagat gtttttgata tctggggcca agggacaaag 360
gttaccgtct cttca                                                  375

<210> 6
<211> 125
<212> PRT
<213> Homo sapiens

<400> 6
Gln Val Gln Leu Gln Gln Ser Gly Gly Gly Leu Val Gln Pro Gly Gly
1               5                   10                  15
Ser Leu Lys Leu Ser Cys Ala Ala Ser Gly Phe Thr Phe Ser Asp Ser
            20                  25                  30
Ala Ile His Trp Val Arg Gln Ala Ser Gly Lys Gly Leu Glu Trp Val
        35                  40                  45
Gly Arg Ile Arg Ser Lys Ala Asn Gly Tyr Ala Thr Ala Tyr Thr Ala
    50                  55                  60
Ser Val Lys Gly Arg Phe Thr Ile Ser Arg Asp Asp Ser Leu Asn Thr
65                  70                  75                  80
Ala Tyr Leu Gln Met Asn Ser Leu Lys Thr Glu Asp Thr Ala Val Tyr
                85                  90                  95
Tyr Cys Thr Arg His Asp Ser Thr Thr Trp Phe Leu Arg Asp Val Phe
            100                 105                 110
Asp Ile Trp Gly Gln Gly Thr Lys Val Thr Val Ser Ser
        115                 120                 125

<210> 7
<211> 327
<212> DNA
<213> Homo sapiens

<400> 7
gacatccagg tgacccagtc tccatcctcc ctgtctgcat ctgtcggaga cagagtcacc 60
atcacttgcc gggcaagtca gggcattgac agagctttag cctggtatca gcagaaatca 120
ggtagacctc ctaagctcct gatctatgat gcctccagtt tagaaagtgg ggtcccatcg 180
aggttcagcg gcagtggatc tgggacagat ttcactctca ccatcagcag cctgcagcct 240
gaagattttg cgacttatta ctgtcaacag tataaaagct accttcgaga gctcactttc 300
ggcggaggga ccaaggtgga gatcaaa                                     327

<210> 8
<211> 109
<212> PRT
<213> Homo sapiens

<400> 8
Asp Ile Gln Val Thr Gln Ser Pro Ser Ser Leu Ser Ala Ser Val Gly
1               5                   10                  15
Asp Arg Val Thr Ile Thr Cys Arg Ala Ser Gln Gly Ile Asp Arg Ala
            20                  25                  30
Leu Ala Trp Tyr Gln Gln Lys Ser Gly Arg Pro Pro Lys Leu Leu Ile
        35                  40                  45

Tyr Asp Ala Ser Ser Leu Glu Ser Gly Val Pro Ser Arg Phe Ser Gly
50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Lys Ser Tyr Leu Arg
                85                  90                  95
Glu Leu Thr Phe Gly Gly Gly Thr Lys Val Glu Ile Lys
            100                 105

<210> 9
<211> 378
<212> DNA
<213> Homo sapiens

<400> 9
caagtgcagc tgttggagtc tggcccagga ctggtgaagc cttcggagac cctgtccctc 60
acctgcactg tctctggtgc ctccatcagc actaagagtt attcctgggg ctggatccgc 120
cagcccccag ggaaggggct ggaatggatt ggtatcgcct acaatagtgg gcgcacctac 180
ttcaatccgt ccctcaagag tcgagtcacc atatccgtgg acacgtccaa gaaccgcttc 240
tccctgcaac ttacctctgt gaccgccgca gacacgtctg catatttctg tgtgagtagt 300
cgtattacaa cattcggagt ggtcactcat tacggtatgg acgtctgggg ccgagggacc 360
acggtcaccg tctcctca                                                378

<210> 10
<211> 126
<212> PRT
<213> Homo sapiens

<400> 10
Gln Val Gln Leu Leu Glu Ser Gly Pro Gly Leu Val Lys Pro Ser Glu
1               5                   10                  15
Thr Leu Ser Leu Thr Cys Thr Val Ser Gly Ala Ser Ile Ser Thr Lys
            20                  25                  30
Ser Tyr Ser Trp Gly Trp Ile Arg Gln Pro Pro Gly Lys Gly Leu Glu
        35                  40                  45
Trp Ile Gly Ile Ala Tyr Asn Ser Gly Arg Thr Tyr Phe Asn Pro Ser
    50                  55                  60
Leu Lys Ser Arg Val Thr Ile Ser Val Asp Thr Ser Lys Asn Arg Phe
65                  70                  75                  80
Ser Leu Gln Leu Thr Ser Val Thr Ala Ala Asp Thr Ser Ala Tyr Phe
                85                  90                  95
Cys Val Ser Ser Arg Ile Thr Thr Phe Gly Val Val Thr His Tyr Gly
            100                 105                 110
Met Asp Val Trp Gly Arg Gly Thr Thr Val Thr Val Ser Ser
        115                 120                 125

<210> 11
<211> 333
<212> DNA
<213> Homo sapiens

<400> 11
cagtctgtgt tgacgcagcc gccctcggtg tcagtggccc caggaacgac ggccagaatt 60
acctgtgcgg ggaacaactt tgcaagtaaa aatgtgcact ggtatcagca gaagccaggc 120
caggcccctg tgctggtcgt ctctgctgat agcgaccggc cctccgaaat ccctgagcga 180
ttttctgcct ccagcactgg gaacacggcc acactgacca tcagcagggt cgacgccggg 240
gatgaggccg actattattg tcaggtttgg gacagtagtc gtgatgatcg ttttgtggtt 300
ttcggcggag gcaccaagct gaccgtccta ggt                               333

<210> 12
<211> 111
<212> PRT
<213> Homo sapiens

<400> 12
Gln Ser Val Leu Thr Gln Pro Pro Ser Val Ser Val Ala Pro Gly Thr
1               5                   10                  15
Thr Ala Arg Ile Thr Cys Ala Gly Asn Asn Phe Ala Ser Lys Asn Val
            20                  25                  30
His Trp Tyr Gln Gln Lys Pro Gly Gln Ala Pro Val Leu Val Val Ser
        35                  40                  45
Ala Asp Ser Asp Arg Pro Ser Glu Ile Pro Glu Arg Phe Ser Ala Ser
    50                  55                  60
Ser Thr Gly Asn Thr Ala Thr Leu Thr Ile Ser Arg Val Asp Ala Gly

65                  70                  75                  80
Asp Glu Ala Asp Tyr Tyr Cys Gln Val Trp Asp Ser Ser Arg Asp Asp
                85                  90                  95
Arg Phe Val Val Phe Gly Gly Gly Thr Lys Leu Thr Val Leu Gly
            100                 105                 110

<210> 13
<211> 375
<212> DNA
<213> Homo sapiens

<400> 13
caggtacagc tgcagcagtc tgggggaggc ttggtcaagc ctggagggtc cctgagactc 60
tcctgtacag cctctggatt catcttcagt gactactata tgagttggat ccgccaggct 120
ccagggaagg gcctggagtg ggtttcatac atgaaaaata gtgatggtag caaatactac 180
gcagactctg tgaagggccg gttcaccatc tccagggaca acgccaagaa ctcattgtat 240
ctgcagatga acagcctgag agccggggac acggctgtct attactgtgt gagagatctt 300
gactactatg ataggagtgg ttaccaccgg tggttcgacc cctggggcca gggaaccctg 360
gtcaccgtct cctca                                                   375

<210> 14
<211> 125
<212> PRT
<213> Homo sapiens

<400> 14
Gln Val Gln Leu Gln Gln Ser Gly Gly Gly Leu Val Lys Pro Gly Gly
1               5                   10                  15
Ser Leu Arg Leu Ser Cys Thr Ala Ser Gly Phe Ile Phe Ser Asp Tyr
            20                  25                  30
Tyr Met Ser Trp Ile Arg Gln Ala Pro Gly Lys Gly Leu Glu Trp Val
        35                  40                  45
Ser Tyr Met Lys Asn Ser Asp Gly Ser Lys Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Gly Asp Thr Ala Val Tyr Tyr Cys
                85                  90                  95
Val Arg Asp Leu Asp Tyr Tyr Asp Arg Ser Gly Tyr His Arg Trp Phe
            100                 105                 110
Asp Pro Trp Gly Gln Gly Thr Leu Val Thr Val Ser Ser
        115                 120                 125

<210> 15
<211> 324
<212> DNA
<213> Homo sapiens

<400> 15
cagtctgtgt tgacgcagcc gccctcagtg tccgtgtccc caggacagac agccagcatc 60
acctgctctg gagataaatt gggacataaa tatgcttgtt ggtatcagca gaagccaggc 120
cagtcccctg tactggtcat ctatcgagat aacaagcggc cctcagggat ccctgagcga 180
ttctctggct ccaactctgg gcacacagcc actctgacca tcagcgggac ccaggctctg 240
gatgaggctg actattactg tcaggcgtgg gacagcagca cccatgtgat attcggcgga 300
ggcaccaagc tgaccgtcct aggt                                         324

<210> 16
<211> 108
<212> PRT
<213> Homo sapiens

<400> 16
Gln Ser Val Leu Thr Gln Pro Pro Ser Val Ser Val Ser Pro Gly Gln
1               5                   10                  15
Thr Ala Ser Ile Thr Cys Ser Gly Asp Lys Leu Gly His Lys Tyr Ala
            20                  25                  30
Cys Trp Tyr Gln Gln Lys Pro Gly Gln Ser Pro Val Leu Val Ile Tyr
        35                  40                  45
Arg Asp Asn Lys Arg Pro Ser Gly Ile Pro Glu Arg Phe Ser Gly Ser
    50                  55                  60
Asn Ser Gly His Thr Ala Thr Leu Thr Ile Ser Gly Thr Gln Ala Leu
65                  70                  75                  80
Asp Glu Ala Asp Tyr Tyr Cys Gln Ala Trp Asp Ser Ser Thr His Val
                85                  90                  95

Ile Phe Gly Gly Gly Thr Lys Leu Thr Val Leu Gly
                100                 105

<210> 17
<211> 21
<212> DNA
<213> Artificial Sequence

<220>
<223> Chemically synthesized primer CVH2

<400> 17
tgccagrtca ccttgargga g                                                21

<210> 18
<211> 21
<212> DNA
<213> Artificial Sequence

<220>
<223> Chemically synthesized primer CVH3

<400> 18
tgcsargtgc agctgktgga g                                                21

<210> 19
<211> 21
<212> DNA
<213> Artificial Sequence

<220>
<223> Chemically synthesized primer CVH4

<400> 19
tgccagstgc agctrcagsa g                                                21

<210> 20
<211> 21
<212> DNA
<213> Artificial Sequence

<220>
<223> Chemically synthesized primer CVH6

<400> 20
tgccaggtac agctgcagca g                                                21

<210> 21
<211> 23
<212> DNA
<213> Artificial Sequence

<220>
<223> Chemically synthesized primer CVH1257

<400> 21
tgccaggtgc agctggtgsa rtc                                              23

<210> 22
<211> 19
<212> DNA
<213> Artificial Sequence

<220>
<223> Chemically synthesized primer C-gamma II

<400> 22
gccaggggga agacsgatg                                                   19

<210> 23
<211> 23
<212> DNA
<213> Artificial Sequence

<220>
<223> Chemically synthesized primer VK1F

<400> 23
      gacatccrgd tgacccagtc tcc                                              23

<210> 24
<211> 23
<212> DNA
<213> Artificial Sequence

<220>
<223> Chemically synthesized primer VK36F

<400> 24
      gaaattgtrw tgacrcagtc tcc                                              23

<210> 25
<211> 23
<212> DNA
<213> Artificial Sequence

<220>
<223> Chemically synthesized primer VK2346F

<400> 25
      gatrttgtgm tgacbcagwc tcc                                              23

<210> 26
<211> 22
<212> DNA
<213> Artificial Sequence

<220>
<223> Chemically synthesized primer VK5F

<400> 26
      gaaacgacac tcacgcagtc tc                                               22

<210> 27
<211> 24
<212> DNA
<213> Artificial Sequence

<220>
<223> Chemically synthesized primer Ck543

<400> 27
      gtttctcgta gtctgctttg ctca                                             24

<210> 28
<211> 23
<212> DNA
<213> Artificial Sequence

<220>
<223> Chemically synthesized primer VL1

<400> 28
      cagtctgtgy tgacgcagcc gcc                                              23

<210> 29
<211> 21
<212> DNA
<213> Artificial Sequence

<220>
<223> Chemically synthesized primer VL2

<400> 29
      cagtctgyyc tgaytcagcc t                                                21

<210> 30
<211> 23

<212> DNA
<213> Artificial Sequence

<220>
<223> Chemically synthesized primer VL3

<400> 30
tcctatgagc tgayrcagcy acc 23

<210> 31
<211> 20
<212> DNA
<213> Artificial Sequence

<220>
<223> Chemically synthesized primer VL1459

<400> 31
cagcctgtgc tgactcaryc 20

<210> 32
<211> 23
<212> DNA
<213> Artificial Sequence

<220>
<223> Chemically synthesized primer VL78

<400> 32
cagdctgtgg tgacycagga gcc 23

<210> 33
<211> 22
<212> DNA
<213> Artificial Sequence

<220>
<223> Chemically synthesized primer VL6

<400> 33
aattttatgc tgactcagcc cc 22

<210> 34
<211> 20
<212> DNA
<213> Artificial Sequence

<220>
<223> Chemically synthesized primer CL2

<400> 34
agctcctcag aggagggygg 20

<210> 35
<211> 32
<212> DNA
<213> Artificial Sequence

<220>
<223> Chemically synthesized primer BsrGIVHF2

<400> 35
aaaatgtaca gtgccagrtc accttgargg ag 32

<210> 36
<211> 32
<212> DNA
<213> Artificial Sequence

<220>
<223> Chemically synthesized primer BsrGIVHF3

<400> 36
aaaatgtaca gtgcsargtg cagctgktgg ag 32

<210> 37
<211> 32
<212> DNA
<213> Artificial Sequence

<220>
<223> Chemically synthesized primer BsrGIVHF4

<400> 37
aaaatgtaca gtgccagstg cagctrcags ag                                32

<210> 38
<211> 32
<212> DNA
<213> Artificial Sequence

<220>
<223> Chemically synthesized primer BsrGIVHF6

<400> 38
aaaatgtaca gtgccaggta cagctgcagc ag                                32

<210> 39
<211> 34
<212> DNA
<213> Artificial Sequence
<220>
<223> Chemically synthesized primer BsrGIVHF1257

<400> 39
aaaatgtaca gtgccaggtg cagctggtgs artc                              34

<210> 40
<211> 21
<212> DNA
<213> Artificial Sequence

<220>
<223> Chemically synthesized primer C gamma ER

<400> 40
gacsgatggg cccttggtgg a                                            21

<210> 41
<211> 35
<212> DNA
<213> Artificial Sequence

<220>
<223> Chemically synthesized primer AgeIVK1F

<400> 41
ttttaccggt gtgacatccr gdtgacccag tctcc                             35

<210> 42
<211> 35
<212> DNA
<213> Artificial Sequence

<220>
<223> Chemically synthesized primer AgeIVK36F

<400> 42
ttttaccggt gtgaaattgt rwtgacrcag tctcc                             35

<210> 43
<211> 35
<212> DNA
<213> Artificial Sequence

<220>
<223> Chemically synthesized primer AgeIVK2346F

<400> 43
      ttttaccggt gtgatrttgt gmtgacbcag wctcc                                35

<210> 44
<211> 34
<212> DNA
<213> Artificial Sequence

<220>
<223> Chemically synthesized primer AgeIVK5F

<400> 44
      ttttaccggt gtgaaacgac actcacgcag tctc                                 34

<210> 45
<211> 33
<212> DNA
<213> Artificial Sequence

<220>
<223> Chemically synthesized primer Sp1KFR4R12

<400> 45
      tttcgtacgt ttgayytcca scttggtccc ytg                                  33

<210> 46
<211> 33
<212> DNA
<213> Artificial Sequence

<220>
<223> Chemically synthesized primer Sp1KFR4R3

<400> 46
      tttcgtacgt ttsakatcca ctttggtccc agg                                  33

<210> 47
<211> 33
<212> DNA
<213> Artificial Sequence

<220>
<223> Chemically synthesized primer Sp1KFR4R4

<400> 47
      tttcgtacgt ttgatctcca ccttggtccc tcc                                  33

<210> 48
<211> 33
<212> DNA
<213> Artificial Sequence

<220>
<223> Chemically synthesized primer Sp1KFR4R5

<400> 48
      tttcgtacgt ttaatctcca gtcgtgtccc ttg                                  33

<210> 49
<211> 32
<212> DNA
<213> Artificial Sequence

<220>
<223> Chemically synthesized primer ApaIVL1

<400> 49
      atatgggccc agtctgtgyt gacgcagccg cc                                   32

<210> 50
<211> 30
<212> DNA
<213> Artificial Sequence

<220>
<223> Chemically synthesized primer ApaIVL2

<400> 50
atatgggccc agtctgyyct gaytcagcct 30

<210> 51
<211> 32
<212> DNA
<213> Artificial Sequence

<220>
<223> Chemically synthesized primer ApaIVL3

<400> 51
atatgggccc agtatgagct gayrcagcya cc 32

<210> 52
<211> 29
<212> DNA
<213> Artificial Sequence

<220>
<223> Chemically synthesized primer ApaIVL1459

<400> 52
atatgggccc agcctgtgct gactcaryc 29

<210> 53
<211> 32
<212> DNA
<213> Artificial Sequence

<220>
<223> Chemically synthesized primer ApaIVL78

<400> 53
atatgggccc agdctgtggt gacycaggag cc 32

<210> 54
<211> 32
<212> DNA
<213> Artificial Sequence

<220>
<223> Chemically synthesized primer ApaIVL6

<400> 54
atatgggccc agttttatgc tgactcagcc cc 32

<210> 55
<211> 29
<212> DNA
<213> Artificial Sequence

<220>
<223> Chemically synthesized primer AvrIIVL1IR

<400> 55
tttcctagga cggtgacctt ggtcccagt 29

<210> 56
<211> 33
<212> DNA
<213> Artificial Sequence

<220>
<223> Chemically synthesized primer AvrIIVL237IR

<400> 56
tttcctagga cggtcagctt ggtscctcck ccg 33

<210> 57
<211> 29

<212> DNA
<213> Artificial Sequence

<220>
<223> Chemically synthesized primer AvrIIVL6IR

<400> 57
tttcctagga cggtcacctt ggtgccact 29

<210> 58
<211> 29
<212> DNA
<213> Artificial Sequence
<220>
<223> Chemically synthesized primer AvrIIVLmixIR
<400> 58
tttcctagga cggtcarctk ggtbcctcc 29

## Claims

1. A fully human monoclonal antibody or fragment thereof that recognizes a component or combination of components of an anthrax exotoxin, wherein said antibody or fragment comprises a heavy chain and a light chain, wherein said heavy chain comprises a heavy chain CDR (complementary determining region) 1, a heavy chain CDR 2, and a heavy chain CDR 3, as set forth in FIG. 5, and wherein said light chain comprises a light chain CDR 1, a light chain CDR 2, and a light chain CDR 3, as set forth in FIG. 6.

2. A fully human monoclonal antibody or fragment thereof according to claim 1, wherein said antibody or fragment comprises a heavy chain variable region having the amino acid sequence shown in FIG.5 and a light chain variable region having the amino acid sequence shown in FIG. 6.

3. A fully human monoclonal antibody or fragment thereof according to claim 1 or 2, wherein said antibody or fragment comprises a heavy chain variable region encoded by the nucleotide sequence shown in FIG. 5 and a light chain variable region encoded by the nucleotide sequence shown in FIG. 6.

4. A fully human monoclonal antibody or fragment thereof according any one of the preceding claims, wherein said antibody or fragment recognizes the 83kD or 63kD form of the protective antigen (PA) portion of anthrax exotoxin.

5. A fully human monoclonal antibody or fragment thereof according to any one of the preceding claims, wherein said antibody or fragment recognizes anthrax exotoxin that is naturally-occurring or synthetic.

6. A fully human monoclonal antibody or fragment thereof according to any one of the preceding claims, wherein said antibody or fragment has a binding affinity of 82.1 pM to 711.0 pM.

7. A pharmaceutical formulation comprising a fully human antibody or fragment thereof according to any one of the preceding claims.

8. An antibody or fragment thereof according to any one of the preceding claims, for use in the treatment of anthrax exposure.

9. An antibody or fragment thereof according to any one of claims 1 to 7, for use against anthrax exposure.

10. A hybridoma comprising the fully human monoclonal antibody or fragment thereof according to any one of claims 1-4.

11. A fully human monoclonal antibody or fragment thereof according to any one of claims 1-4, wherein the fully human monoclonal antibody or fragment thereof is expressed as a recombinant protein on CHO cells.

12. A stable CHO cell line expressing a fully human antibody or fragment thereof according to any one of claims 1-4.

13. An *in vitro* method for inhibiting the assembly of the protective antigen (PA) of an anthrax exotoxin, the method comprising providing a fully human monoclonal antibody or fragment thereof according to any one of claims 1-4 to disrupt said assembly.

14. A method of identifying the presence of anthrax exotoxin in a sample, comprising: contacting at least a portion of said sample with the fully human antibody or fragment thereof according to any one of claims 1-4; and determining binding of anthrax exotoxin with said antibody, wherein said binding is an indicator of the presence anthrax in said sample.

15. A kit to identify the presence of anthrax exotoxin in a sample, comprising: the fully human monoclonal antibody or fragment thereof according to any one of claims 1-4; and an assay system to determine the binding of anthrax exotoxin with said antibody, wherein said binding is an indicator of the presence anthrax in said sample.
